# EUROPEAN PATENT APPLICATION

(11) **EP 4 501 201 A1**
(43) Date of publication of application: **05.02.2025**
(21) Application number: 22933170.7
(22) Date of filing: 16.12.2022
(51) Int. Cl.: A61B 1/00

(54) **MEDICAL ENDOSCOPE DEVICE AND MEDICAL ENDOSCOPE CAMERA SYSTEM**

(30) Priority: 25.03.2022 WO PCT/CN2022/083135
(71) Applicant: Shenzhen Mindray Bio-Medical Electronics Co., Ltd., Shenzhen, Guangdong 518057 (CN)
(72) Inventor: ZENG, Qiang, Shenzhen, Guangdong 518057 (CN); HU, Lian, Shenzhen, Guangdong 518057 (CN); SHI, Qiangyong, Shenzhen, Guangdong 518057 (CN); XU, Tao, Shenzhen, Guangdong 518057 (CN)
(74) Representative: KIPA AB
(86) International application number: PCT/CN2022/139782
(87) International publication number: WO 2023/179121

(57) **Abstract**

Provided are a medical endoscope device and a medical endoscope camera system. The medical endoscope device comprises an optical assembly, an image sensor assembly, a driving apparatus, an operating member, and a detection apparatus. A trigger area of the detection apparatus can reflect or transmit detection light to form reflected light or transmitted light having a difference, and the reflected light or the transmitted light having a difference can generate electrical signals having a difference, so as to represent the rotation angle and/or direction of the operating member, so as to control the driving apparatus to adjust the optical path distance between the optical assembly and the image sensor assembly to achieve focusing.

## Description

### TECHNICAL FIELD

The disclosure relates to a technical field of medical device, and in particular, to a medical endoscope device and an imaging system for medical endoscope.

### BACKGROUND

Rigid endoscopes, such as cystoscope and hysteroscope, are mainly used for diagnosis and/or treatment of lesions in natural cavities and punctured cavities in superficial and shallow parts of a human body. Rigid endoscopes cannot be bent during operation.

A rigid endoscope mainly includes an imaging head, a light source, a light guide beam, a rigid endoscope, an optical bayonet, an imaging head host, and a display. The imaging head includes an optical component, and an operation member (usually called a hand wheel), which is configured to drive the optical component to perform focusing.

Chinese patent application with an application number 201580000890.9 discloses an operation apparatus for medical device, which apparatus can be applied to an endoscope imaging head. In which, when a handwheel is used for focusing, a rotation angle of the handwheel is detected by a magnetic encoder to achieve a focusing function.

### SUMMARY

In an embodiment, a medical endoscope device is provided, including:
an insertion portion, which includes a front end and a rear end; wherein the front end of the insertion portion is capable of being inserted into a region of a patient to be observed, so as to acquire an image light which is reflected and/or excited by the region of the patient to be observed; and
an imaging head, which includes:
   an optical component, wherein the optical component and the rear end of the insertion portion are optically butted;
   an image sensor component, wherein the optical component and the image sensor component are movable relative to each other; the optical component is configured to illuminate the image sensor component with the image light which is acquired by the insertion portion; the image sensor component is configured to obtain the image light and generate an image signal according to the image light, wherein the image signal is used to generate a visible image of the region of the patient to be observed;
   a driving apparatus, which is connected with the optical component and/or the image sensor component, and is configured to drive at least one of the optical component and the image sensor component to move, so as to adjust a distance of an optical path between the optical component and the image sensor component;
   a rotatable operation member; and
   a detection apparatus, which includes an optical triggering member and an optical detection member, wherein at least one of the optical triggering member and the optical detection member, and the operation member are linked together, so as to move together, and a rotation of the operation member is capable of enabling a relative rotation between the optical triggering member and the optical detection member;
   the optical triggering member is provided with a triggering area;
   the optical detection member is configured to emit a detection light and illuminate the triggering area with the detection light; the triggering area is configured to reflect or transmit the detection light, wherein during the rotation of the operation member, different positions of the triggering area are illuminated by the detection light; the optical detection member is further configured to receive the detection light which is reflected or transmitted by the triggering area, and generate different electrical signals based on the reflected or transmitted detection light; the driving apparatus is configured to drive at least one of the optical component and the image sensor component to move according to the different electrical signals, so as to adjust the distance of the optical path between the optical component and the image sensor component to achieve focusing.

In an embodiment, the different electrical signals are capable of at least being used to determine a rotation angle of the operation member.

In an embodiment, reflection or transmission of the detection light at different positions of the triggering area is capable of forming different reflected lights or different transmitted lights; the optical detection member is further configured to receive the different reflected lights or the different transmitted lights, and generate the different electrical signals based on the different reflected lights or the different transmitted lights.

In an embodiment, the triggering area includes a plurality of sub-triggering areas which are arranged along a circumferential direction, wherein adjacent sub-triggering areas have different light reflectivity or different light transmissivity.

In an embodiment, each sub-triggering area has a same width along the circumferential direction, and/or each pair of adjacent sub-triggering areas has a same space.

In an embodiment, light reflectivity or light transmissivity of the plurality of sub-triggering areas increases or decreases along the circumferential direction.

In an embodiment, the triggering area includes a first sub-triggering area and a second sub-triggering area which are alternately arranged, wherein the first sub-triggering area and the second sub-triggering area have different light reflectivity or different light transmissivity.

In an embodiment, the optical detection member is specifically configured to emit a first detection light and a second detection light, the electrical signals include a first electrical signal and a second electrical signal, the different positions of the triggering area are simultaneously illuminated with the first detection light and the second detection light; reflection or transmission of the first detection light by the triggering area forms a first reflected light or a first transmitted light, and reflection or transmission of the second detection light by the triggering area forms a second reflected light or a second transmitted light; wherein the optical detection member is specifically configured to receive the first reflected light or the first transmitted light and generate the first electrical signal, and to receive the second reflected light or the second transmitted light and generate a second electrical signal, wherein the first electrical signal and/or the second electrical signal are/is used to calculate a rotation angle of the operation member, and the first electrical signal and the second electrical signal are used to jointly determine a rotation direction of the operation member.

In an embodiment, when the first sub-triggering area is illuminated with the first detection light, the second sub-triggering area is illuminated with the second detection light; and when the second sub-triggering area is illuminated with the first detection light, the first sub-triggering area is illuminated with the second detection light.

In an embodiment, the first electrical signal includes a first pulse signal, the second electrical signal includes a second pulse signal, and a difference in pulse phase exists between the first pulse signal and the second pulse signal.

In an embodiment, the difference in pulse phase between the first pulse signal and the second pulse signal is 90°.

In an embodiment, the optical detection member includes a first optical detection member and a second optical detection member; wherein the first optical detection member is configured to emit the first detection light, receive the first reflected light or the first transmitted light, and generate the first electrical signal; the second optical detection member is configured to emit the second detection light, receive the second reflected light or the second transmitted light, and generate the second electrical signal.

In an embodiment, the triggering area has gradually changing light reflectivity or gradually changing light transmissivity along a circumferential direction.

In an embodiment, the gradually changing light reflectivity or the gradually changing light transmissivity is linearly changing light reflectivity or linearly changing light transmissivity.

In an embodiment, the optical triggering member is provided with a side flat surface, a central axis of the relative rotation between the optical triggering member and the optical detection member is perpendicular to the side flat surface, the triggering area is arranged on the side flat surface; or
the optical triggering member is provided with a cylindrical surface, a central axis of the cylindrical surface overlaps with a central axis of the relative rotation between the optical triggering member and the optical detection member, and the triggering area is arranged on the cylindrical surface.

In an embodiment, the optical detection member is provided with an emitting end and a receiving end, wherein the triggering area is configured to reflect the detection light, wherein the emitting end and the receiving end are located at a same side of the optical triggering member; and/or
the triggering area is configured to transmit the detection light, wherein the emitting end and the receiving end are located at respective sides of the optical triggering member.

In an embodiment, an environment surrounding an optical path of the optical component and an environment surrounding an optical path of the detection apparatus are isolated from each other.

In an embodiment, a lens barrel is further included, the optical component is arranged inside the lens barrel, the environment surrounding the optical path of the optical component is located at an inner side of the lens barrel, the environment surrounding the optical path of the detection apparatus is located at an outer side of the lens barrel.

In an embodiment, the operation member is located at the outer side of the lens barrel, the operation member is capable of rotating relative to the lens barrel, the operation member and/or the lens barrel are/is provided with an annular groove, and the detection apparatus is located inside the annular groove.

In an embodiment, the operation member is provided with an annular groove, the optical triggering member is located at a side wall of the annular groove along an axial direction, and the optical detection member is arranged at the lens barrel.

In an embodiment, the annular groove has an axial opening, wherein the axial opening is provided with a first sealing member.

In an embodiment, the imaging head is provided with a sealed cavity, wherein the detection apparatus is located inside the sealed cavity.

In an embodiment, the optical component and the image sensor component are located inside the sealed cavity.

In an embodiment, the imaging head is provided with a sealed cavity, the optical triggering member is located outside the sealed cavity, and the optical detection member is located inside the sealed cavity.

In an embodiment, a second sealing member is provided between the optical triggering member and the optical detection member, the second sealing member surrounds a part of the sealed cavity, wherein the detection light is capable of passing through the second sealing member.

In an embodiment, the second sealing member is a transparent or translucent structure.

In an embodiment, the second sealing member is a structure which is capable of allowing a designated light to pass therethrough, wherein the detection light includes the designated light.

In an embodiment, a medical endoscope device is provided, including:
an insertion portion, which includes a front end and a rear end; wherein the front end of the insertion portion is capable of being inserted into a region of a patient to be observed, so as to acquire an image light which is reflected and/or excited by the region of the patient to be observed; and
an imaging head, which includes:
   an optical component, wherein the optical component and the rear end of the insertion portion are optically butted;
   an image sensor component, wherein the optical component and the image sensor component are movable relative to each other; the optical component is configured to illuminate the image sensor component with the image light which is acquired by the insertion portion; the image sensor component is configured to obtain the image light and generate an image signal according to the image light, wherein the image signal is used to generate a visible image of the region of the patient to be observed;
   a driving apparatus, which is connected with the optical component and/or the image sensor component, and is configured to drive at least one of the optical component and the image sensor component to move, so as to adjust a distance of an optical path between the optical component and the image sensor component;
   a rotatable operation member; and
   a fixation member, wherein the operation member is rotatable relative to the fixation member, one of the operation member and the fixation member is provided with a triggering area; and
   a detection apparatus, which includes an optical detection member, wherein the optical detection member is arranged at the other of the operation member and the fixation member which is not provided with the triggering area, and a rotation of the operation member is capable of enabling a relative rotation between the triggering area and the optical detection member;
   the optical detection member is configured to emit a detection light and illuminate the triggering area with the detection light; the triggering area is configured to reflect or transmit the detection light, wherein during the rotation of the operation member, different positions of the triggering area are illuminated by the detection light; the optical detection member is further configured to receive the detection light which is reflected or transmitted by the triggering area, and generate different electrical signals based on the reflected or transmitted detection light; the driving apparatus is configured to drive at least one of the optical component and the image sensor component to move according to the different electrical signals, so as to adjust the distance of the optical path between the optical component and the image sensor component to achieve focusing.

In an embodiment, the different electrical signals are capable of at least being used to determine a rotation angle of the operation member.

In an embodiment, reflection or transmission of the detection light at different positions of the triggering area is capable of forming different reflected lights or different transmitted lights; the optical detection member is further configured to receive the different reflected lights or the different transmitted lights, and generate the different electrical signals based on the different reflected lights or the different transmitted lights.

In an embodiment, the triggering area includes a plurality of sub-triggering areas which are arranged along a circumferential direction, wherein adjacent sub-triggering areas have different light reflectivity or different light transmissivity.

In an embodiment, light reflectivity or light transmissivity of the plurality of sub-triggering areas increases or decreases along the circumferential direction.

In an embodiment, the triggering area includes a first sub-triggering area and a second sub-triggering area which are alternately arranged, wherein the first sub-triggering area and the second sub-triggering area have different light reflectivity or different light transmissivity.

In an embodiment, the optical detection member is specifically configured to emit a first detection light and a second detection light, the electrical signals include a first electrical signal and a second electrical signal, the different positions of the triggering area are simultaneously illuminated with the first detection light and the second detection light; reflection or transmission of the first detection light by the triggering area forms a first reflected light or a first transmitted light, and reflection or transmission of the second detection light by the triggering area forms a second reflected light or a second transmitted light; wherein the optical detection member is specifically configured to receive the first reflected light or the first transmitted light and generate the first electrical signal, and to receive the second reflected light or the second transmitted light and generate a second electrical signal, wherein the first electrical signal and/or the second electrical signal are/is used to calculate a rotation angle of the operation member, and the first electrical signal and the second electrical signal are used to jointly determine a rotation direction of the operation member.

In an embodiment, when the first sub-triggering area is illuminated with the first detection light, the second sub-triggering area is illuminated with the second detection light; and when the second sub-triggering area is illuminated with the first detection light, the first sub-triggering area is illuminated with the second detection light.

In an embodiment, the first electrical signal includes a first pulse signal, the second electrical signal includes a second pulse signal, and a difference in pulse phase exists between the first pulse signal and the second pulse signal.

In an embodiment, the triggering area has gradually changing light reflectivity or gradually changing light transmissivity along a circumferential direction.

In an embodiment, the triggering area has linearly changing light reflectivity or linearly changing light transmissivity along the circumferential direction.

In an embodiment, a medical endoscope device is provided, including:
a rotatable operation member; and
a detection apparatus, which includes an optical triggering member and an optical detection member;
at least one of the optical triggering member and the optical detection member, and an operation member are linked together, so as to move together and a rotation of the operation member is capable of enabling a relative rotation between the optical triggering member and the optical detection member;
the optical triggering member is provided with a triggering area;
the optical detection member is configured to emit a detection light and illuminate the triggering area with the detection light; the triggering area is configured to reflect or transmit the detection light, wherein during the rotation of the operation member, different positions of the triggering area are illuminated by the detection light; the optical detection member is further configured to receive the detection light which is reflected or transmitted by the triggering area, and generate different electrical signals based on the reflected or transmitted detection light; wherein the different electrical signals are used for focusing of the medical endoscope device.

In an embodiment, the different electrical signals are capable of at least being used to determine a rotation angle of the operation member.

In an embodiment, reflection or transmission of the detection light at different positions of the triggering area is capable of forming different reflected lights or different transmitted lights; the optical detection member is further configured to receive the different reflected lights or the different transmitted lights, and generate the different electrical signals based on the different reflected lights or the different transmitted lights.

In an embodiment, the triggering area includes a plurality of sub-triggering areas which are arranged along a circumferential direction, wherein adjacent sub-triggering areas have different light reflectivity or different light transmissivity.

In an embodiment, light reflectivity or light transmissivity of the plurality of sub-triggering areas increases or decreases along the circumferential direction.

In an embodiment, the triggering area includes a first sub-triggering area and a second sub-triggering area which are alternately arranged, wherein the first sub-triggering area and the second sub-triggering area have different light reflectivity or different light transmissivity.

In an embodiment, the optical detection member is specifically configured to emit a first detection light and a second detection light, the electrical signals include a first electrical signal and a second electrical signal, the different positions of the triggering area are simultaneously illuminated with the first detection light and the second detection light; reflection or transmission of the first detection light by the triggering area forms a first reflected light or a first transmitted light, and reflection or transmission of the second detection light by the triggering area forms a second reflected light or a second transmitted light; wherein the optical detection member is specifically configured to receive the first reflected light or the first transmitted light and generate the first electrical signal, and to receive the second reflected light or the second transmitted light and generate a second electrical signal, wherein the first electrical signal and/or the second electrical signal are/is used to calculate a rotation angle of the operation member, and the first electrical signal and the second electrical signal are used to jointly determine a rotation direction of the operation member.

In an embodiment, when the first sub-triggering area is illuminated with the first detection light, the second sub-triggering area is illuminated with the second detection light; and when the second sub-triggering area is illuminated with the first detection light, the first sub-triggering area is illuminated with the second detection light.

In an embodiment, the first electrical signal includes a first pulse signal, the second electrical signal includes a second pulse signal, and a difference in pulse phase exists between the first pulse signal and the second pulse signal.

In an embodiment, the triggering area has gradually changing light reflectivity or gradually changing light transmissivity along a circumferential direction.

In an embodiment, the triggering area has linearly changing light reflectivity or linearly changing light transmissivity along the circumferential direction.

In an embodiment, a medical endoscope device is provided, including:
a rotatable operation member; and
a fixation member, wherein the operation member is rotatable relative to the fixation member, one of the operation member and the fixation member is provided with a triggering area; and
a detection apparatus, which includes an optical detection member, wherein the optical detection member is arranged at the other of the operation member and the fixation member which is not provided with the triggering area, and a rotation of the operation member is capable of enabling a relative rotation between the triggering area and the optical detection member;
the optical detection member is configured to emit a detection light and illuminate the triggering area with the detection light; the triggering area is configured to reflect or transmit the detection light; wherein the optical detection member is further configured to receive the detection light which is reflected or transmitted by the triggering area, and generate different electrical signals based on the reflected or transmitted detection light; wherein the different electrical signals are used for focusing of the medical endoscope device.

In an embodiment, the different electrical signals are capable of at least being used to determine a rotation angle of the operation member.

In an embodiment, reflection or transmission of the detection light at different positions of the triggering area is capable of forming different reflected lights or different transmitted lights; the optical detection member is further configured to receive the different reflected lights or the different transmitted lights, and generate the different electrical signals based on the different reflected lights or the different transmitted lights.

In an embodiment, the triggering area includes a plurality of sub-triggering areas which are arranged along a circumferential direction, wherein adjacent sub-triggering areas have different light reflectivity or different light transmissivity.

In an embodiment, light reflectivity or light transmissivity of the plurality of sub-triggering areas increases or decreases along the circumferential direction.

In an embodiment, the triggering area includes a first sub-triggering area and a second sub-triggering area which are alternately arranged, wherein the first sub-triggering area and the second sub-triggering area have different light reflectivity or different light transmissivity.

In an embodiment, the optical detection member is specifically configured to emit a first detection light and a second detection light, the electrical signals include a first electrical signal and a second electrical signal, the different positions of the triggering area are simultaneously illuminated with the first detection light and the second detection light; reflection or transmission of the first detection light by the triggering area forms a first reflected light or a first transmitted light, and reflection or transmission of the second detection light by the triggering area forms a second reflected light or a second transmitted light; wherein the optical detection member is specifically configured to receive the first reflected light or the first transmitted light and generate the first electrical signal, and to receive the second reflected light or the second transmitted light and generate a second electrical signal, wherein the first electrical signal and/or the second electrical signal are/is used to calculate a rotation angle of the operation member, and the first electrical signal and the second electrical signal are used to jointly determine a rotation direction of the operation member.

In an embodiment, when the first sub-triggering area is illuminated with the first detection light, the second sub-triggering area is illuminated with the second detection light; and when the second sub-triggering area is illuminated with the first detection light, the first sub-triggering area is illuminated with the second detection light.

In an embodiment, the first electrical signal includes a first pulse signal, the second electrical signal includes a second pulse signal, and a difference in pulse phase exists between the first pulse signal and the second pulse signal.

In an embodiment, the triggering area has gradually changing light reflectivity or gradually changing light transmissivity along a circumferential direction.

In an embodiment, the triggering area has linearly changing light reflectivity or linearly changing light transmissivity along the circumferential direction.

In an embodiment, an imaging system for medical endoscope is provided, including a light source, a light guide beam, an imaging head host, a display and the above-mentioned medical endoscope device; wherein the light source is connected with the insertion portion through the light guide beam, the illumination light and/or the excitation light which are/is emitted by the light source pass(es) through the light guide beam and the insertion portion in sequence to illuminate the region of the patient to be observed; the imaging head host is in signal connection with the imaging head of the medical endoscope device and the display respectively; the imaging head host is configured to obtain the image signal, and output the image signal to the display for displaying after processing.

According to the medical endoscope device and the imaging system for medical endoscope of the above-mentioned embodiments, since the medical endoscope device is provided with an optical detection apparatus which is capable of emitting and receiving detection lights, and a triggering area of the detection apparatus is capable of reflecting or transmitting the detection lights, so as to form different reflected lights or transmitted lights. Such different reflected lights or transmitted lights are capable of generating different electrical signals to characterize a rotation angle and/or a rotation direction of the operation member, thereby controlling the driving apparatus to adjust a distance of an optical path between an optical component and an image sensor component, so as to achieve focusing. The rotation angle and the rotation direction of the operation member are firstly adjusted and then the driving apparatus is used to electrically drive the focusing. As the operation member belongs to indirect focusing and is a non-direct driving member, which removes a rotation restriction of the operation member and allows the operation member to rotate arbitrarily, thus greatly improving feel of use and increasing a range of focusing.

### BRIEF DESCRIPTION OF THE DRAWINGS

FIG. 1 is a structural diagram of a medical endoscope device in an embodiment.
FIG. 2 is a structural diagram of an imaging head in an embodiment.
FIG. 3 is a partial axial cross-sectional diagram of a medical endoscope device in an embodiment.
FIG. 4 is a partial three-dimensional cross-sectional diagram of a medical endoscope device in an embodiment.
FIG. 5 is a partial enlarged diagram of position A in FIG. 3.
FIG. 6 is a structural diagram of an optical triggering member (grating code wheel) in an embodiment.
FIG. 7 is a structural diagram of an optical detection member (photoelectric encoding chip) in an embodiment.
FIG. 8 is a structural diagram of an optical triggering member (grating code wheel) in an embodiment.
FIG. 9 is a partial schematic diagram of a grating code wheel in an embodiment.
FIG. 10 is a schematic diagram of a first pulse signal and a second pulse signal in an embodiment.
FIG. 11 is a structural diagram of a detection apparatus in an embodiment.
FIG. 12 is a structural diagram of an optical detection member (photoelectric switch) in an embodiment.
FIG. 13 is a schematic diagram of a sealed cavity of a medical endoscope device in an embodiment.
FIG. 14 is a schematic diagram of an embodiment in which an optical triggering member and an optical detection member are respectively located inside and outside a sealed cavity;
FIG. 15 is a structural diagram of an imaging system for medical endoscope in an embodiment.

The reference numbers in the drawings are as follows.
1-Optical component, 11-fixed optical component, 12-adjustable optical component;
2-Image sensor component, 21-housing, 22-sensor;
3-driving apparatus, 31-motor, 32-screw rod, 33-slider;
4-Operation member, 41-annular groove, 42-first sealing member, 43-second sealing member;
5-detection apparatus, 51-optical triggering member, 51a-side flat surface, 51b-cylindrical surface, 511-triggering area, 5111-first sub-triggering area, 5112-second sub-triggering area, 52-optical detection member, 52a-emitting end, 52b-receiving end, 521-first optical detection member, 522-second optical detection member, 53-mounting seat, 54-signal connection line, 55-annular wheel, 551-convex strip;
6-Handle;
7-lens barrel (fixation member), 71-threading hole, 72-light shielding member;
8-front cover;
A-sealed cavity;
1000-imaging system for medical endoscope, 10-light source, 20-light guide beam, 30-insertion portion, 40-optical bayonet, 50-imaging head, 81-communication cable, 60-imaging head host, 70-display, 82-video connection cable, 100-region of patient to be observed.

### DESCRIPTION OF THE PREFERRED EMBODIMENTS

Detection apparatus may include various forms, such as a contact detection apparatus and a non-contact detection apparatus.

A contact detection apparatus includes a conductive wheel and a probe. The conductive wheel is arranged at an operation member and is provided with a conductive area and a non-conductive area which are alternatively arranged, or with a high resistance area and a low resistance area which are alternatively arranged. When the conductive wheel rotates with the operation member, a conductive needle contacts the conductive area and the non-conductive area which are alternatively arranged, so as to form a pulse signal. The pulse signal can be used to calculate an operation amount of the operation member and a focusing parameter of an image sensor component can be adjusted based on the operation amount.

A non-contact detection apparatus includes a photoelectric detection apparatus and a detection apparatus of magnetic induction. The photoelectric detection apparatus detects by detecting lights, and the detection apparatus of magnetic induction detects changes in a magnetic field.

The photoelectric detection includes an optical triggering member and an optical detection member. The optical triggering member is arranged at the operation member and has a triggering area. The optical detection member is capable of emitting a detection light and receiving the detection light which is reflected or transmitted by the optical triggering member. The optical detection member is capable of emitting detection lights to different positions of the triggering area of the optical triggering member. The different positions of the triggering area of the optical triggering member are configured to reflect or transmit the detection lights, so as to form different reflected lights or transmitted lights. When the optical triggering member is rotating with the operation member, the optical detection member emits detection lights to different positions of the triggering area and obtains different reflected lights or transmitted lights, so as to form different electrical signals (such as pulse signals). The operation amount of the operation member can be calculated through the electrical signals.

The scheme of the detection apparatus of magnetic induction can be found in Chinese patent application 201580000890.9.

The detection apparatus of this disclosure adopts a non-contact photoelectric detection apparatus. Compared with the contact detection apparatus, the non-contact detection apparatus has advantages of higher detection accuracy and longer service life. The contact detection apparatus has a shorter service life and is also prone to false touches. Compared with the detection apparatus of magnetic induction, the photoelectric detection apparatus has advantages of low interference and high detection accuracy. Detection apparatus of magnetic inductions are easily affected by environmental magnetic fields. In particular, even a small environmental magnetic field can cause a large detection error.

The detection apparatus of this disclosure can be arranged inside a sealed cavity, or the optical detection member of the detection apparatus, which member is involved in electrical connection, can be arranged in a sealed cavity, so that the medical endoscope device equipped with the detection apparatus can be sterilized by high temperature and immersion.

The medical endoscope device of this disclosure may be an imaging head for medical endoscope. The imaging head is of a miniaturized structure and can be held by a doctor for use. Setting a detection apparatus inside the miniaturized imaging head requires optimization and improvement for the existing component structure. In this disclosure, an accommodation cavity is provided between the operation member (hand wheel) and the lens barrel, a mounting groove is provided on a surface of the operation member, which surface faces the lens barrel, or a mounting groove is provided on a surface of the lens barrel, which surface faces the operation member, and the mounting grooves provided by the lens barrel and the operation member surround the accommodation cavity. The detection apparatus is arranged inside the accommodation cavity between the operation member and the lens barrel. While outer sizes of the operation member and the lens barrel remain unchanged, a space for accommodating the detection apparatus is increased, that is, while keeping the medical endoscope device small in size, detections for a rotation angle and a rotation direction of the operation member can be achieved.

This disclosure is further described in detail below through specific embodiments in conjunction with the accompanying drawings. Similar elements in different embodiments are labelled with similar element numbers. In the following embodiments, many details are described to enable this disclosure to be better understood. However, those skilled in the art can easily realize that some of the features may be omitted in different situations, or may be replaced by other elements, materials, or methods. In some cases, some operations related to this disclosure are not shown or described in the description. This is to avoid the core part of this disclosure being overwhelmed by excessive description. For those skilled in the art, it is not necessary to describe these related operations in detail. They can fully understand the related operations based on the description in this disclosure and the general technical knowledge in the field.

In addition, the features, operations, or characteristics described in the description may be combined in any appropriate manner to form various implementations. At the same time, the steps, or actions in description for the method may also be swapped or adjusted in order, in a manner that is obvious to those skilled in the art. Therefore, the various sequences in the description and the drawings are only for the purpose of clearly describing a certain embodiment, and does not mean to be a necessary sequence unless otherwise specified that a certain sequence must be followed.

The serial numbers assigned to components in this disclosure, such as "first", "second", etc., are only to distinguish the objects described and do not have any order or technical meaning. The terms "connection" and "coupling" mentioned in this disclosure include direct and indirect connections (couplings) unless otherwise specified. A front end in this disclosure refers to an end which is adjacent to the patient, and a rear end refers to an end, which is away from the patient.

In an embodiment, a medical endoscope device is provided. The medical endoscope device may include an imaging head and an insertion portion. The imaging head is driven indirectly to achieve focusing. An operation member is used as a reference target. A detection apparatus and a driving apparatus are added. The detection apparatus monitors an operation amount of the operation member by the user, and then controls the driving apparatus to drive the optical component to move for focusing according to the operation amount.

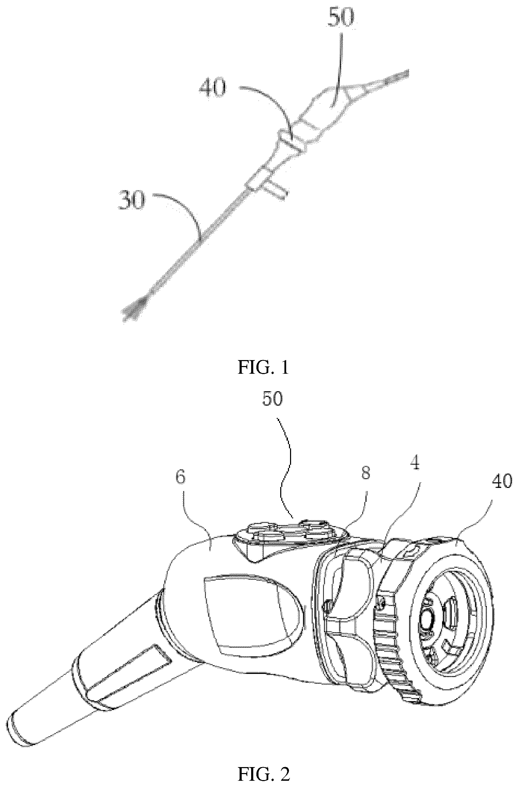

Please refer to FIG. 1, FIG. 2 and FIG. 3, the medical endoscope device of this embodiment mainly includes an insertion portion 30 and an imaging head 50.

The insertion portion 30 can be a rigid endoscope, and the insertion portion 30 has a front end and a rear end. The insertion portion 30 has an illumination channel and an imaging channel. The illumination channel can include a light guide beam, such as optical fibre, and the imaging channel can include an imaging component, such as imaging lenses.

The front end of the insertion portion 30 is capable of being inserted into a region of a patient to be observed, and illuminating the region of the patient to be observed with an illumination light and/or excitation light through the illumination channel. The region of the patient to be observed is capable of reflecting the illumination light, or the region of the patient to be observed is capable of being excited by the excitation light, so as to generate an excitation light (such as fluorescence). The insertion portion 30 is further configured to acquire the illumination light and/or excitation light, which are/is reflected by the region of the patient to be observed. The image light acquired by the insertion portion 30 may be transmitted to the imaging head 50 through an imaging channel.

The imaging head 50 is capable of obtaining the image light which is acquired by the insertion portion 30 and generate an image signal (electrical signal) based on the image light. The image signal is used to generate a visible image of the region of the patient to be observed. The imaging head 50 is capable of transmitting the image signal to an imaging head host. The imaging head host processes the image signal to generate a visible image of the region of the patient to be observed, and outputs the visible image to the display 70 for displaying.

In other embodiments, it can also be considered that the medical endoscope device only includes an imaging head 50, the front end of the imaging head 50 is connected with an insertion portion 30, and the rear end of the imaging head 50 is connected with an imaging head host to perform imaging observation on the region of the patient to be observed.

In an embodiment, the imaging head 50 mainly includes an optical component 1, an image sensor component 2, a driving apparatus 3, an operation member 4, a detection apparatus 5, a handle 6 and a lens barrel 7.

The handle 6 mainly includes an outer housing part. The handle 6 further includes a front end and a rear end, and a cavity provided inside the handle 6. The front end of the handle 6 can be detachably connected with the rear end of the insertion portion 30, and the rear end of the handle 6 can be connected with a cable, and the cable can be plugged into the imaging head host. Buttons may be provided on an outer side of the handle 6, and the buttons may be used for imaging control and adjustment.

The lens barrel 7 is located inside the cavity of the handle 6, and a front end of the lens barrel 7 is exposed from the handle 6 for connecting with an optical bayonet 40. The optical bayonet 40 can fix the front end of the lens barrel 7 and the rear end of the insertion portion 30 together, wherein the optical bayonet 40 can realize a detachable connection between the insertion portion 30 and the imaging head 50. The rear end of the lens barrel 7 is connected with the image sensor component 2.

The optical component 1 is configured to obtain the image light which is acquired by the insertion portion 30, and provide a transmission optical path for the image light. The optical component 1 is located inside the lens barrel 7, and a front end of the optical component 1 is used for optical butting with a rear end of the insertion portion 30. The optical butting can be a direct contact butting of the two components, or a spaced butting, while ensuring that optical axes of the front and rear components are aligned, so that the imaging light which is acquired by the insertion portion 30 can be transmitted to the optical component 1.

The optical component 1 includes a fixed optical component 11 and an adjustable optical component 12. The fixed optical component 11 is fixedly installed inside the lens barrel 7. The fixed optical component 11 includes a fixed lens seat and a fixed lens. One or more fixed lenses are installed inside the fixed lens seat. The adjustable optical component 12 is axially movable inside the lens barrel 7. The adjustable optical component 12 can axially move relative to the fixed optical component 11 to achieve focusing and imaging. The adjustable optical component 12 includes an adjustable lens seat and an adjustable lens. One or more adjustable lenses are installed inside the adjustable lens seat. The adjustable lens seat and the adjustable lens are an integral structure and moved axially together. In other embodiments, only the adjustable lens may be moved, and the adjustable lens seat is a fixed member.

The image sensor component 2 is installed inside the cavity of the handle 6. The image sensor component 2 is configured to receive the image light which is transmitted by the optical component 1, and convert the image light into an image signal (electrical signal). The image sensor component 2 is also in signal connection with the driving apparatus 3 and the detection apparatus 5.

A control circuit may be provided at the rear end of the image sensor component 2, so as to obtain an electrical signal which is generated by the detection apparatus 5 through detecting a rotation of the operation member 4, and so as to control the driving apparatus 3 to drive the adjustable optical component 12 in the optical component 1 to move according to the electrical signal to achieve focusing. In other embodiments, the control circuit may also be arranged at an imaging head host, and the imaging head host transmits a control signal to the imaging head, so as to control the driving apparatus 3 to achieve focusing.

The image sensor component 2 mainly includes a housing 21 and an image sensor 22. A front end of the housing 21 is connected with the lens barrel 7 through a front cover 8, and a rear end of the lens barrel 7 abuts against the housing 21. The image sensor 22 is installed inside the housing 21 through a mounting structure such as a bracket, and the image sensor 22 is aligned with the fixed optical component 11 and the adjustable optical component 12 along the optical axis, so that the image sensor 22 can be illuminated with the image light, which is transmitted by the fixed optical component 11 and the adjustable optical component 12. In an embodiment, the control circuit may also be installed inside the housing 21 and located at the rear end of the image sensor 22, and the image sensor 22 is in signal connection with the control circuit. In some embodiments, the control circuit may be arranged at an independent circuit board, and the control circuit may include a control chip and related circuit module(s).

The driving apparatus 3 is installed inside the cavity of the handle 6. The driving apparatus 3 includes a motor 31, a screw rod 32 and a slider 33. The driving apparatus 3 is arranged inside the housing 21 and is connected with the housing 21 via a mounting structure, such as a fixing frame. The motor 31 may be a stepping motor. The screw rod 32 is connected with an output shaft of the motor 31. The slider 33 has a threaded hole and a through-hole. The slider 33 is axially movably connected with the screw rod 32 through the threaded hole. The slider 33 is connected with the adjustable optical component 12 .

A forward rotation and a reverse rotation of the motor 31 are used to drive the slider 33 to move forward or backward in the axial direction, so that the driving apparatus 3 can drive the adjustable optical component 12 to move precisely in the axial direction.

The applicant of this application filed, on December 29, 2021, a Chinese patent application with an application number 202111633040.6 and named "Endoscopic Imaging head and Endoscopic Imaging System", which records a variety of drive structure schemes for focusing an endoscopic imaging head, and these schemes can be applied to the embodiments of this application as specific implementation schemes of the driving apparatus 3; therefore, the entire content of this application is incorporated herein by reference.

In other embodiments, the driving apparatus 3 includes a linear motor, and an output end of the linear motor is directly connected with the adjustable optical component 12, wherein this arrangement can also achieve precise axial adjustment of the adjustable optical component 12.

In other embodiments, the driving apparatus 3 is located inside the cavity of the handle 6, and the driving apparatus 3 is connected with the lens barrel 1, which plays a role of supporting and fixing the driving apparatus 3.

In other embodiments, the optical component 1 only includes a fixed optical component 11, the image sensor component 2 includes a movable image sensor 22, which is axially movably arranged inside the housing 21. The driving apparatus 3 is arranged inside the cavity of the handle 6, or is arranged inside the housing 21, and the driving apparatus 3 is configured to drive the image sensor to move, so as to adjust a distance of an optical path between the fixed optical component 11 and the image sensor 22 to achieve focusing.

In other embodiments, the driving apparatus 3 may include two driving apparatuses, one driving apparatus is connected with the adjustable optical component 12, and the other driving apparatus is connected with the image sensor 22. The image sensor 22 is axially movable along the optical axis, and is arranged inside the housing 21. The two driving apparatuses 3 can be driven simultaneously or separately to adjust the distance of the optical path between the optical component 1 and the image sensor component 2 to achieve focusing.

In this embodiment, the motor 31 may be electrically connected with a control circuit at the rear end of the image sensor 22, wherein the control circuit is configured to control a number of rotation(s) and a direction of rotation(s) of the motor 31, thereby controlling a moving direction and a moving distance of the adjustable optical component 12 .

The operation member 4 may be an annular hand wheel. The operation member 4 is a focusing operation member. The operation member 4 rotates according to an operation of a user, so as to indirectly adjust an imaging focal length of the optical component 1, thereby achieving focusing.

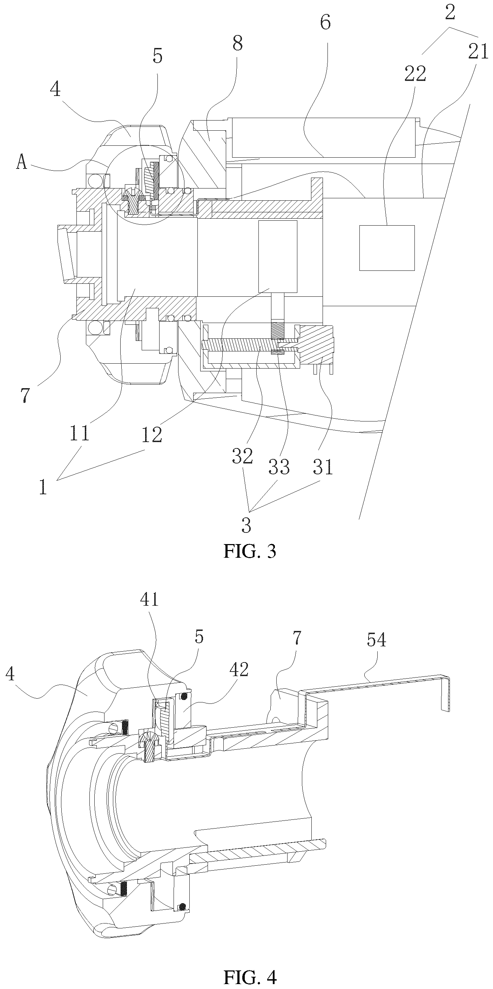

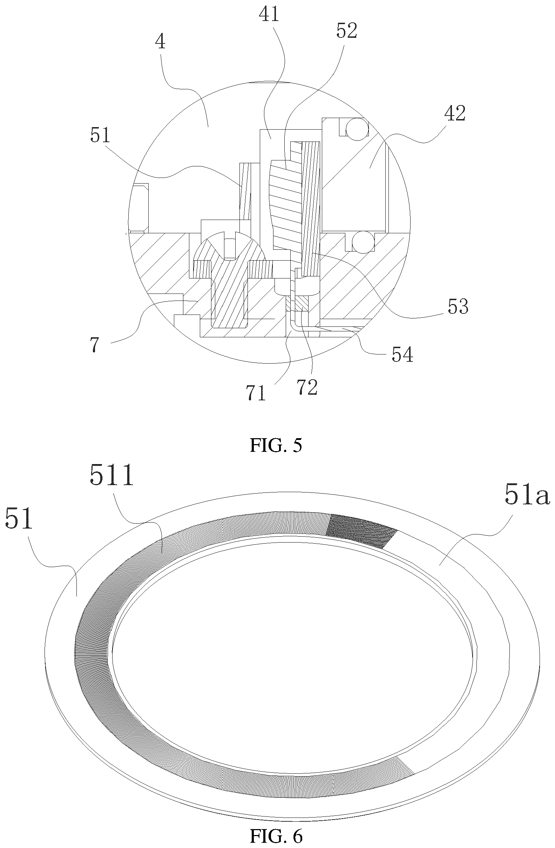

Please refer to FIG. 4 and FIG. 5, the operation member 4 is rotatably mounted at the lens barrel 7, and the operation member 4 can rotate forward and backward for arbitrarily 360° relative to the lens barrel 7. The operation member 4 is an annular structure and is provided with an annular groove 41. The annular groove 41 is provided with a radial opening which faces the lens barrel 7. The annular groove 41 is configured to accommodate the detection apparatus 5. The detection apparatus 5 is configured to detect an operation amount of the operation member 4, that is, to detect a rotation angle and a rotation direction of the operation member 4.

The annular groove 41 of the operation member 4 has an axial opening, which faces the rear end of the operation member 4. A first sealing member 42 is installed at the axial opening. The first sealing member 42 seals the detection apparatus 5 inside the annular groove 41, so as to protect the detection apparatus 5 and avoid light interference of external light on the detection apparatus 5.

The lens barrel 7 isolates an environment surrounding an optical path of the optical component 1 from an environment surrounding an optical path of the detection apparatus 5, such that the environment surrounding the optical path of the optical component 1 and the environment surrounding the optical path of the detection apparatus 5 do not interfere with each other, which is beneficial to improving imaging quality and detection accuracy. The environment surrounding the optical path of the optical component 1 refers to a space where the imaging light is transmitted, and the environment surrounding the optical path of the detection apparatus 5 refers to a space where the detection light exists. The two spaces are physically isolated from each other, so that the imaging light and the detection light cannot be integrated with each other.

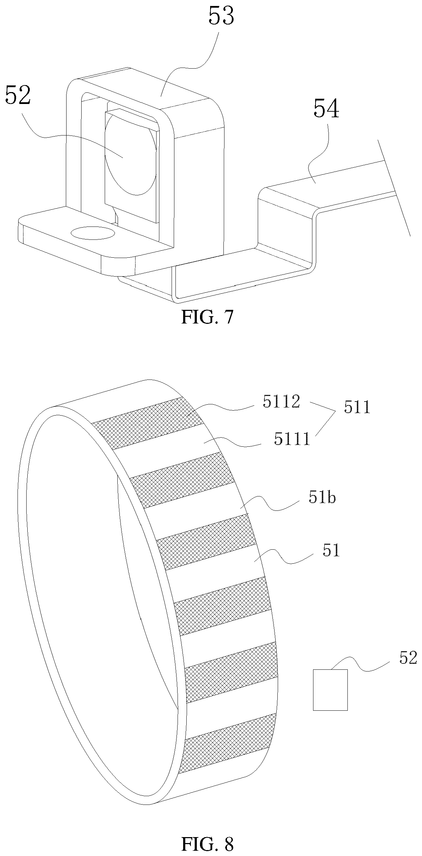

Please refer to FIG. 6 and FIG. 7. In an embodiment, the detection apparatus 5 includes an optical triggering member 51 and an optical detection member 52. The optical triggering member 51 is fixed to a side wall along an axial direction inside the annular groove 41 of the operation member 4 by bonding or the likes. The other side of the optical triggering member 51 relative to the bonding surface is a side flat surface 51a, wherein a central axis of a relative rotation between the optical triggering member 51 and the optical detection member 52 is perpendicular to the side flat surface 51a. A triggering area 511 is provided on the side flat surface 51a. The triggering area 511 can be an annular area. The triggering area 511 is provided with a first sub-triggering area 5111 and a second sub-triggering area 5112 which are alternately arranged along a circumferential direction of the annular area. The first sub-triggering area 5111 and the second sub-triggering area 5112 have different light reflectivity. For example, the first sub-triggering area 5111 can achieve nearly 100% total reflection, and the second sub-triggering area 5112 can achieve nearly 0% zero reflection (or full absorption) or 50% semi-reflection.

The detection apparatus 5 can be a photoelectric encoding detection apparatus, the optical triggering member 51 can be a grating code wheel with a ring structure, the first sub-triggering area 5111 can be a glossy area to achieve higher reflectivity; the second sub-triggering area 5112 can be a hollow area without reflection, that is, light directly passes through the second sub-triggering area. In actual situations, the detection light passes through the hollow second sub-triggering area 5112 and is illuminated onto the operation member 4. The corresponding area on the operation member 4 is arranged as a low-reflection surface, such as a diffuse reflection surface, so that light reflectivity formed by the detection light, which light passes through the second sub-triggering area 5112, is also smaller than the light reflectivity of the first sub-triggering area 5111, thereby achieving detection.

Wherein, the operation member 4 can be made of rubber, the grating code wheel is made of metal, grating holes of the grating code wheel are the second sub-triggering area 5112, a metal area between the grating holes of the grating code wheel is the first sub-triggering area 5111, wherein the first sub-triggering area 5111 and the second sub-triggering area 5112 are located on a circular flat surface. A light-absorbing layer is attached to a surface of the operation member 4, which surface is connected with the grating code wheel, and a reflective layer with holes is attached to the other side of the grating code wheel, which side is opposite to the operation member 4. This arrangement can also realize the first sub-triggering area 5111 and the second sub-triggering area 5112. The materials of the operation member 4 and the grating code wheel can be selected arbitrarily.

The first sub-triggering area 5111 and the second sub-triggering area 5112 can have central angles with a same span, that is, a sectorial area of the grating hole and an area between the grating holes are equal in shape and size. The first sub-triggering area 5111 and the second sub-triggering area 5112 have a same width in the circumferential direction, so that when the detection light is illuminated along the circumferential path of the triggering area 511, it can pass through the first sub-triggering area 5111 and the second sub-triggering area 5112 to travel a same trajectory length, which is conducive to improving the accurate detection of the rotation angle and the rotation direction.

In other embodiments, the optical triggering member 51 can be a wheel without holes, the first sub-triggering area 5111 and the second sub-triggering area 5112 are surfaces with different reflectivity, such as the first sub-triggering area 5111 and the second sub-triggering area 5112 are coated with reflective coatings of different types or densities, which can also enable the first sub-triggering area 5111 and the second sub-triggering area 5112 to reflect different reflected lights.

Please refer to FIG. 8. In other embodiments, the optical triggering member 51 is a ring-shaped structure or a tubular structure. The optical triggering member 51 has a cylindrical surface 51b, which is a circumferential outer surface of the optical triggering member 51. A central axis of the cylindrical surface overlaps with a central axis of the relative rotation between the optical triggering member 51 and the optical detection member 52. The triggering area 511 is arranged on the cylindrical surface 51b, and the triggering area 511 includes a first sub-triggering areas 5111 and a second sub-triggering areas 5112 which are alternatively arranged with each other. The optical detection member 52 is arranged on an outer side of the ring-shaped structure or the cylindrical structure, which can also detect the rotation of the operation member 4.

In an embodiment, the optical detection member 52 includes an emitting end 52a and a receiving end 51b, wherein the emitting end 52a and the receiving end 51b are arranged side by side at a same side of the optical triggering member 51, the emitting end 52a is configured to emit the detection light to illuminate the triggering area 511 of the optical triggering member 51, and the receiving end 51b is configured to receive different detection lights which are reflected by the triggering area 511. The optical detection member 52 generates different electrical signals according to the received different detection lights, and transmits the generated electrical signals to the control circuit. The control circuit calculates the rotation angle and the rotation direction of the operation member 4 according to the different electrical signals, and controls the driving apparatus 3 to drive the adjustable optical component 12 to move, so as to achieve focusing and zooming. The electrical signal may be a pulse signal, and the control circuit may calculate the rotation angle of the operation member 4 according to a pulse number.

In an embodiment, the triggering area 511 is provided with first sub-triggering areas 5111 and second sub-triggering areas 5112 which are alternately arranged. Since all the first sub-triggering areas 5111 have same first reflectivity and all the second sub-triggering areas 5112 have same second reflectivity, wherein the first reflectivity is different from the second reflectivity. Therefore, only the rotation angle of the operation member 4 can be detected by using a single-channel detection light. In order to detect the rotation direction of the operation member 4, the optical detection member 52 needs to emit two paths of detection lights, that is, the optical detection member 52 is specifically configured to emit a first detection light and a second detection light. Different sub-triggering areas are illuminated with the first detection light and the second detection light. For example, when the first sub-triggering area 5111 is illuminated with the first detection light, the second sub-triggering area 5112 is illuminated with the second detection light. Alternatively, when the second sub-triggering area 5112 is illuminated with the first detection light, the first sub-triggering area 5111 is illuminated with the second detection light. The triggering areas 511 with different reflectivity are illuminated with the first detection light and the second detection light, so that the first detection light and the second detection light simultaneously generate a first reflected light and a second reflected light which are different from each other. The first reflected light and the second reflected light which are different from each other can be used to calculate the rotation direction of the operation member 4.

The optical detection member 52 includes two emitting ends 52a and two receiving ends 52b, wherein the two emitting ends 52a and the two receiving ends 52b form two groups of one-to-one corresponding transceiver. For example, the optical detection member 52 includes a first emitting end and a second emitting end, and also includes a first receiving end and a second receiving end. The first emitting end corresponds to the first receiving end, and the second emitting end corresponds to the second receiving end.

The optical detection member 52 may include a photoelectric encoding chip, which includes a light-emitting LED light source and an optical signal detection circuit. The light-emitting LED is the emitting end 52a of the photoelectric encoding chip, and the optical signal detection circuit is provided with a photosensitive receiving tube, which is the receiving end 52b.

The light-emitting LED can specifically form two paths of emitted light through a lens, so as to form a first emitting end and a second emitting end. The first emitting end is configured to emit a first detection light, and the second emitting end is configured to emit a second detection light. Two photosensitive receiving tubes are arranged at the optical signal detection circuit, and respectively used as a first receiving end and a second receiving end. The first receiving end is configured to receive the first detection light which is reflected by the first sub-triggering area 5111 of the grating code wheel, and the photoelectric encoding chip generates a first electrical signal based on the emitted and received first detection light. The second receiving end is configured to receive the second detection light which is reflected by the second sub-triggering area 5112 of the grating code wheel, and the photoelectric encoding chip generates a second electrical signal based on the emitted and received second detection light.

The first electrical signal includes a first pulse signal, and the second electrical signal includes a second pulse signal. The first pulse signal and the second pulse signal are pulse signals with a phase difference, wherein the phase difference can be used to determine the rotation direction of the operation member 4.

The first emitting end and the second emitting end of the photoelectric encoding chip are arranged side by side in a radial direction, and the first receiving end and the second receiving end of the photoelectric encoding chip are also arranged side by side in the radial direction, so that the first detection light and the second detection light, which are simultaneously emitted by the photoelectric encoding chip, respectively illuminate different circumferential positions of the grating code wheel, and a phase difference exists between pulse signals which are respectively formed by the first detection light and the second detection light, wherein the first detection light and the second detection light are respectively received by the first receiving end and the second receiving end of the photoelectric encoding chip.

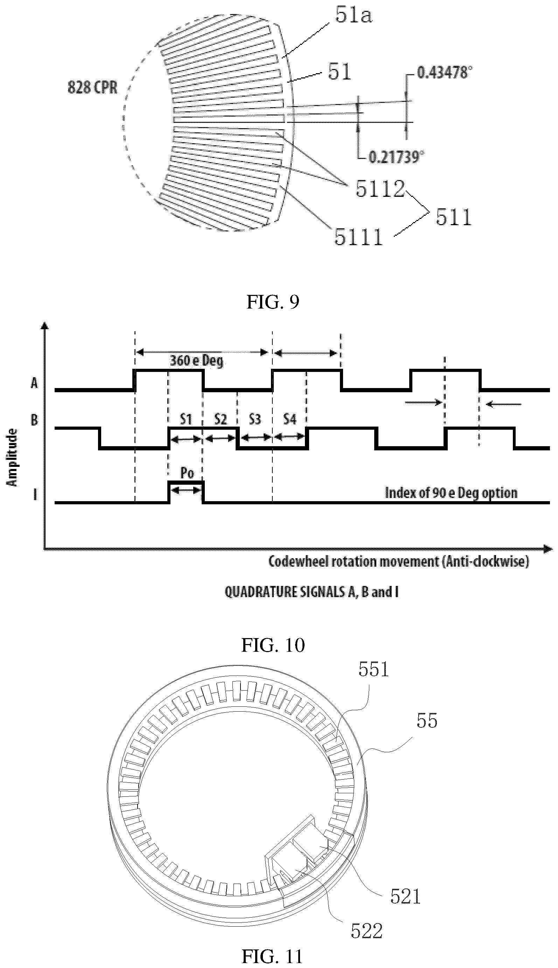

Please refer to FIG. 9. In an embodiment, burning of the grating code wheel has certain requirements, which stipulate that N (approximately equal to 11.7) grating lines (second reflective area 512) can be burned per millimetre. The grating line has a sectorial area, and a central angle of the sectorial area is 0.21739°. A central angle of the sectorial area between grating lines is also 0.21739°, that is, a central angle of the sectorial area which is surrounded by the first sub-triggering area 5111 and the second sub-triggering area 5112 is 0.43478°. The grating code wheel with a radius of R can be burned with a total of grating lines CPR=2*3.148*R*N, that is, every time the grating code wheel rotates one circle, the first pulse signal and the second pulse signal can output CPR pulses respectively, and an angular resolution of each pulse is r=360/CPR. Therefore, the rotation angle of the grating code wheel can be calculated according to the number of outputted pulses (either the first pulse signal or the second pulse signal), that is, the rotation angle of the operation member 4 can be calculated. Assuming that the number of outputted pulses is n, the rotation angle between the grating code wheel and the operation member 4 is w=n*r=n*360/(2*3.148*R*N).

In an embodiment, a preset radial space is provided between the first emitting end and the second emitting end of the photoelectric encoding chip, so that the generated first pulse signal and the generated second pulse signal have a phase difference of 90°. The phase difference between the first pulse signal and the second pulse signal may also be 60° or other phase difference degrees.

Please refer to FIG. 10. When the grating code wheel rotates counterclockwise with the operation member 4, a phase of the first pulse signal is 90° advanced than a phase of the second pulse signal. When the grating code wheel rotates clockwise with the operation member 4, the phase of the second pulse signal is 90° advanced than the phase of the first pulse signal. In FIG. 10, A represents the first pulse signal and B represents the second pulse signal. The control circuit can determine the rotation direction of the operation member 4 according to the phase difference between the first pulse signal and the second pulse signal.

In other implementations, the photoelectric encoding chip has a processing module, and the photoelectric encoding chip calculates the rotation angle and the rotation direction of the operation member 4 through the detected first pulse signal and second pulse signal.

In other embodiments, the photoelectric encoding chip further has a control module. The photoelectric encoding chip is directly connected with the driving apparatus 3. The photoelectric encoding chip controls the driving apparatus 3 to drive the optical component 1 to focus, according to the calculated rotation angle and rotation direction of the operation member 4.

Please refer to FIG. 5 and FIG. 7. In this embodiment, a mounting seat 53 is mounted on an outside of the lens barrel 7 by a screw, and the optical detection member 52 is mounted on the mounting seat 53. The mounting seat 53 is an L-shaped structure, and has a radial mounting surface. The photoelectric encoding chip is located on the mounting surface of the mounting seat 53, so that the photoelectric encoding chip is axially aligned with the grating code wheel.

The lens barrel 7 is also provided with a threading hole 71, and the photoelectric encoding chip is connected with the control circuit at the rear end of the image sensor 22 through a signal connection line 54. One end of the signal connection line 54 is connected with the photoelectric encoding chip, and the other end of the signal connection line 54 passes through the threading hole 71 and extends into the housing 21 to be connected with the control circuit.

In an embodiment, a light shielding member 72 is further provided inside the threading hole 71. The light shielding member 72 is a rubber elastic plug or a cap. The light shielding member 72 blocks the threading hole 71 to prevent light from entering the lens barrel 7. The light shielding member 72 isolates the environment surrounding the optical path in the optical component 1 from the environment surrounding the optical path of the detection apparatus 5, thereby preventing the detection light of the detection apparatus 5 from affecting the imaging light which is transmitted in the optical component 1 .

In an embodiment, the environment surrounding the optical path of the detection apparatus 5 is arranged inside a sealed space, which can not only enable the environment surrounding the optical path of the detection apparatus 5 to be isolated from the environment surrounding the optical path of the optical path assembly 1, but also is conducive to the high-temperature disinfection of the imaging head 50. During the high-temperature disinfection process, a sterilized air or liquid does not enter the detection apparatus 5 in the operation member 4. The sealed space can provide high-temperature disinfection protection for the detection apparatus 5, so that the imaging head 50 can satisfy requirements of clinical repeated use.

In an embodiment, the threading hole 71 is not provided at the lens barrel 7, but is provided at the housing 21. The signal connection line 54 extends into the housing 21 from the threading hole at the housing 21 and is connected with the control circuit, which also realize connection between the photoelectric encoding chip and the control circuit, and can also isolate the environment surrounding the optical path in the optical component 1 and the environment surrounding the optical path of the detection apparatus 5 from each other.

In an embodiment of this disclosure, the optical detection member 52 can be configured to emit a detection light, such as laser and visible light, and the triggering area 511 on the optical triggering member 51 is configured to reflect or transmit the detection light, such as laser and visible light, so as to realize the detection of the rotation angle and rotation direction of the operation member 4, and realize focusing.

In an embodiment, the optical detection member 52 can be configured to emit ultrasonic wave(s), and the optical triggering member 51 is configured to reflect the ultrasonic wave(s). The triggering area 511 on the optical triggering member 51 can reflect the ultrasonic wave(s) to form ultrasonic waves of different intensities. The optical detection member 52 can generate different electrical signals by acquiring ultrasonic waves of different intensities, so as to detect the forwarding angle and rotation direction of the operation member 4 and realize focusing.

In an embodiment, the optical detection member 52 can be configured to emit an excitation light, and the triggering area 511 of the optical triggering member 51 is coated with a material that can be excited to emit fluorescence when illuminated with the excitation light, and different positions on the triggering area 511 are provided with materials of different densities, so that when the optical detection member 52 is configured to emit an excitation light to illuminate different positions of the triggering area 511, the different positions of the triggering area 511 are excited to emit fluorescence with different energy densities. The optical detection member 52 can generate different electrical signals by acquiring the fluorescence with different energy densities, so as to realize the detection of the forwarding angle and rotation direction of the operation member 4, and realize focusing.

In an embodiment of this disclosure, since a detection apparatus 5 is provided, wherein the detection apparatus 5 is configured to detect the rotation direction and the rotation angle of the operation member 4, and then the optical component 1 is driven by the driving apparatus 3 to achieve focusing. The operation member 4 is a non-direct driving member, such that the rotation restriction of the operation member 4 is released. In this way, the operation member 4 can be rotated arbitrarily within 360° to indirectly drive the focusing, which greatly improves feel of use and increases the focusing range.

In an embodiment, the optical detection member 52 includes two photoelectric encoding chips, each of which has an emitting end and a receiving end. The two photoelectric encoding chips are respectively arranged at different circumferential positions, so that the two photoelectric encoding chips output two pulse signals with a phase difference. In this way, the rotation angle and rotation direction of the operation member 4 can also be calculated.

Specifically, the optical detection member 52 includes a first optical detection member 521 and a second optical detection member 522. The first optical detection member 521 is a first photoelectric encoding chip, and the second optical detection member 522 is a second photoelectric encoding chip. The first photoelectric encoding chip and the second photoelectric encoding chip are arranged side by side relative to the grating code wheel.

The first photoelectric encoding chip includes a first emitting end and a first receiving end. The first emitting end and the first receiving end of the first photoelectric encoding chip are located at a same side of the grating code wheel, the first emitting end of the first photoelectric encoding chip is configured to emit the first detection light, the first receiving end of the first photoelectric encoding chip is configured to receive the first detection light which is reflected from the triggering area, and the first photoelectric encoding chip is configured to generate a first pulse signal according to the emitted and received first detection light. The second photoelectric encoding chip includes a second emitting end and a second receiving end. The second emitting end and the second receiving end of the second photoelectric encoding chip are located at a same side of the grating code wheel, the second emitting end of the second photoelectric encoding chip is configured to emit the second detection light, the second receiving end of the second photoelectric encoding chip is configured to receive the second detection light which is reflected from the triggering area, and the second photoelectric encoding chip is configured to generate a second pulse signal according to the emitted and received second detection light.

In other embodiments, the optical detection member 52 includes a photoelectric encoding chip, which includes two emitting ends and two receiving ends. One photoelectric encoding chip simultaneously emits a first detection light and a second detection light, and simultaneously receives a first reflected light and a second reflected light. In this way, the rotation angle and rotation direction of the operation member 4 can also be calculated.

In some embodiments, the detection apparatus 5 implements a detection in a transmitting manner. The optical detection member 52 is configured to emit a detection light, and the triggering area 511 on the optical triggering member 51 is configured to transmit the detection light. Different positions on the triggering area 511 have different light transmissivity to form different transmitted lights. The optical detection member 52 is further configured to receive the different transmitted lights to generate different electrical signals. The different electrical signals can also be used to calculate the rotation angle and the rotation direction of the operation member 4.

If the detection apparatus 5 uses two photoelectric switches for detection, the two photoelectric switches are configured to respectively generate a first pulse signal and a second pulse signal with a phase difference. In this way, the rotation angle, and the rotation direction of the operation member 4 can also be calculated.

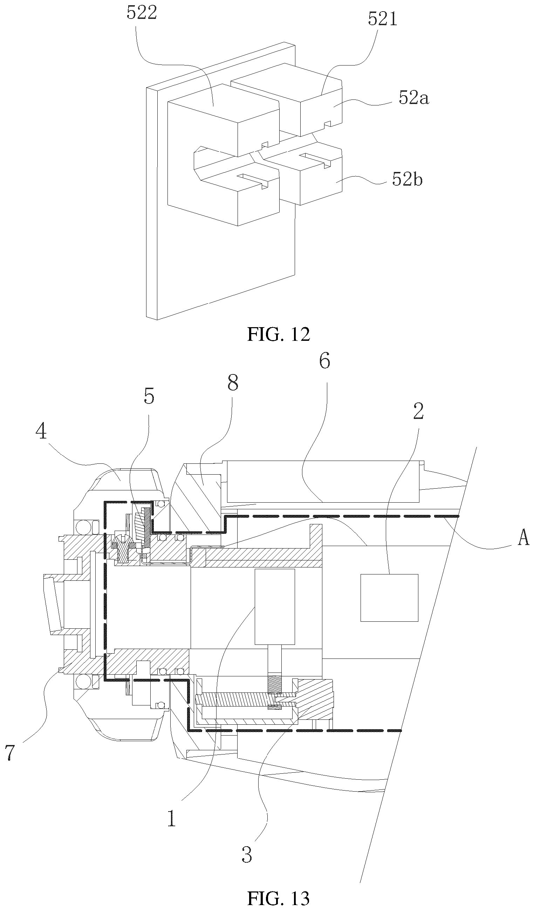

Please refer to FIGS. 11 and 12, the detection apparatus 5 includes an annular wheel 55, a first photoelectric switch 56, and a second photoelectric switch 57, wherein the annular wheel 55 is an optical triggering member 51, the first photoelectric switch 56 is a first optical detection member 521, and the second photoelectric switch 57 is a second optical detection member 522.

A plurality of convex strips 551 are uniformly distributed on a circumference of the annular wheel 55. The convex strips 551 are located in an annular flat surface. The convex strips 551 form first sub-triggering areas 5111. Gap areas between the convex strips 551 form second sub-triggering areas 5112. The gap areas between the convex strips 551, and the convex strips 551 form the first sub-triggering areas 5111 and the second sub-triggering areas 5112 which are alternately arranged. The first sub-triggering areas 5111 and the second sub-triggering areas 5112 are located in one flat surface.

The first sub-triggering area 5111 has light transmissivity of 0%, that is, directly blocks the detection light, and the second sub-triggering area 5112 has a light transmissivity of 100%.

The first sub-triggering area 5111 and the second sub-triggering area 5112 can also be both physical structures. The first sub-triggering area 5111 and the second sub-triggering area 5112 are glass materials with different transmissivity. For example, the first sub-triggering area 5111 has a light transmissivity of 10%, and the second sub-triggering area 5112 has a light transmissivity of 90%. The first sub-triggering area 5111 and the second sub-triggering area 5112 can also form transmitted lights with different transmissivity.

The first photoelectric switch 56 and the second photoelectric switch 57 are arranged side by side along the circumferential direction. The first photoelectric switch 56 and the second photoelectric switch 57 respectively have an emitting end 52a and a receiving end 52b, wherein the emitting end 52a and the receiving end 52b form a U-shaped structure with a main body of the switch. The convex strips 551 of the annular wheel 55 are arranged inside the U-shaped grooves of the first photoelectric switch 56 and of the second photoelectric switch 57. The emitting end 52a and the receiving end 52b are respectively located on respective sides of the annular wheel 55 (optical triggering member 51). The convex strip 551 of the annular wheel 55 is equivalent to being configured to cut the optical signal in the U-shaped groove of the first photoelectric switch 56 and the second photoelectric switch 57, so that the first photoelectric switch 56 and the second photoelectric switch 57 respectively form an alternating state of on-off-on-off, thereby forming a pulse signal.

The first photoelectric switch 56 includes a first emitting end and a first receiving end. The first emitting end and the first receiving end of the first photoelectric switch 56 are respectively located on respective sides of the convex strip 551. The first emitting end of the first photoelectric switch 56 is configured to emit a first detection light. The first receiving end of the first photoelectric switch 56 is configured to receive the first detection light which is transmitted by a first triggering area. The first photoelectric switch generates a first pulse signal according to the emitted and received first detection light.

The second photoelectric switch 57 includes a second emitting end and a second receiving end. The second emitting end and the second receiving end of the second photoelectric switch 57 are respectively located on respective sides of the convex strip 551. The second emitting end of the second photoelectric switch 57 is configured to emit a second detection light. The second receiving end of the second photoelectric switch 57 is configured to receive the second detection light which is transmitted by a second triggering area. The second photoelectric switch generates a second pulse signal according to the emitted and received second detection light.

Two photoelectric switches are configured to replace the photoelectric encoding chip in the above embodiment. The rotation angle of the operation member 4 can also be calculated by switching-on and switching-off states of the photoelectric switches. The arrangement of the two photoelectric switches can also calculate the rotation direction of the operation member 4 based on the phase difference, thereby realizing focusing of the optical component 1.

In an embodiment, a plurality of sub-triggering areas may be arranged on the triggering area 511, and the plurality of sub-triggering areas are sequentially distributed along the circumferential direction of the triggering area 511, wherein adjacent sub-triggering areas have different light reflectivity or different light transmissivity. For example, the light reflectivity of the plurality of sub-triggering areas is sequentially 10%, 40%, 30%, 70%, 50%..., and it is sufficient to ensure that adjacent sub-triggering areas have different light reflectivity, so that when two adjacent sub-triggering areas are illuminated with the detection light, different reflected lights are formed, and then different electrical signals can be obtained. In this way, the detection of the rotation angle and direction of the operation member 4 can also be realized. Alternatively, the triggering area 511 is arranged to have transmissivity of 10%, 40%, 30%, 70%, 50% ..., and different transmitted lights can also be obtained, that is, different electrical signals can be obtained, such that the rotation angle and direction of the operation member 4 can also be detected.

In an embodiment, the light reflectivity, or the light transmissivity of the plurality of sub-triggering areas on the triggering area 511 is gradually increased or decreased. For example, the light reflectivity of the plurality of sub-triggering areas is 10%, 20%, 30%, 40%, 50%..., in sequence. Adjacent sub-triggering areas also have different light reflectivity, which can generate different reflected lights and obtain different electrical signals. In this way, the detection of the rotation angle of the operation member 4 can also be realized. Moreover, since the light reflectivity of the plurality of sub-triggering areas decreases in sequence or changes in a regular manner, the light reflectivity increases in sequence in the clockwise direction, and the light reflectivity decreases in sequence in the counterclockwise direction, so that when one detection light is used to detect the rotation angle of the operation member 4, the rotation direction of the operation member 4 can also be confirmed. When the light reflectivity increases, the operation member 4 rotates clockwise; when the light reflectivity decreases, the operation member 4 rotates counterclockwise. Of course, whether the increase and decrease of the light reflectivity corresponds to the clockwise rotation of or the counterclockwise rotation of the operation member 4, can be set according to requirements. Similarly, the light transmissivity of the plurality of sub-triggering areas can be set as 10%, 20%, 30%, 40%, 50% ... in sequence, so as to detect the rotation angle and rotation direction of the operation member 4 by one detection light.

In an embodiment, the triggering area 511 can be provided with light reflectivity or transmissivity that gradually changes along the circumferential direction. There are no obvious sub-triggering areas which are divided on the triggering area 511. The triggering area 511 is provided with gradually changing light reflectivity or transmissivity with a more microscopic difference. For example, the triggering area 511 is provided with linearly changing light reflectivity or linearly changing light transmissivity, for example, 1%, 2%, 3%, 4%, 5%... In this structure, an area with 1% linearly changing light reflectivity or transmissivity can span a smaller circumferential angle, so that the triggering area 511 has a higher detection accuracy.

In an embodiment, the plurality of sub-triggering areas on the triggering area 511 can be sub-triggering areas which are separated from each other, and areas between the sub-triggering areas can be non-responsive to the detection light. For example, when a reflection detection is adopted, the areas between the sub-triggering areas can be a hollow area or a pure black area, and the hollow area or the pure black area can be regarded as not responding to the detection light. The detection light can generate intermittent electrical signals, which can also be used to detect the rotation angle and rotation direction of the operation member 4. The plurality of sub-triggering areas may have a same space to improve the detection accuracy of the rotation angle.

In an embodiment, the optical detection member 52 is fixed at the fixation member (7, 21), the fixation member may be the lens barrel 7, and the fixation member (7, 21) may also be the housing 21. The optical triggering member 51 is mounted at the operation member 4, so that the optical triggering member 51 can rotate relative to the optical detection member 52 which is fixed at the fixation member (7, 21). In this way, the detection of the rotation angle and rotation direction of the operation member 4 can also be realized.

In an embodiment, the operation member 4 can also be linked with the optical detection member 52, the optical triggering member 51 is fixed at the fixation member (7, 21), the optical triggering member 51 can be fixed at the lens barrel 7, and the optical triggering member 51 can also be fixed at the housing 21. The optical detection member 52 can rotate relative to the optical triggering member 51, which is fixed at the fixation member (7, 21). In this way, the detection of the rotation angle and rotation direction of the operation member 4 can also be realized.

In an embodiment, the optical triggering member 51 or the optical detection member 52 is directly fixed at the operation member 4, so that the optical detection member 52 can drive the optical triggering member 51 or the optical detection member 52 to rotate. The optical triggering member 51 or the optical detection member 52 can also be connected with the operation member 4 through a transmission component. For example, the optical triggering member 51 or the optical detection member 52 is installed at a rotation shaft, and the operation member 4 drives the rotation shaft to rotate through a transmission component such as a gear set, thereby driving the optical triggering member 51 or the optical detection member 52 to rotate. In this way, the linkage between the operation member 4 and the optical triggering member 51 or the optical detection member 52 can also be realized.

In an embodiment, the medical endoscope device may only include an imaging head 50, and the medical endoscope device does not include an insertion portion 30. A front end of the imaging head 50 may be detachably connected with A rear end of the insertion portion 30 via an optical bayonet 40.

In an embodiment, the medical endoscope device may also only include a driving apparatus 3, an operation member 4 and a detection apparatus 5. The medical endoscope device can be used as a detection apparatus. The detection apparatus can be installed in combination at an existing imaging head. The operation member 4 can indirectly drive the driving apparatus 3 inside the imaging head to drive focusing.

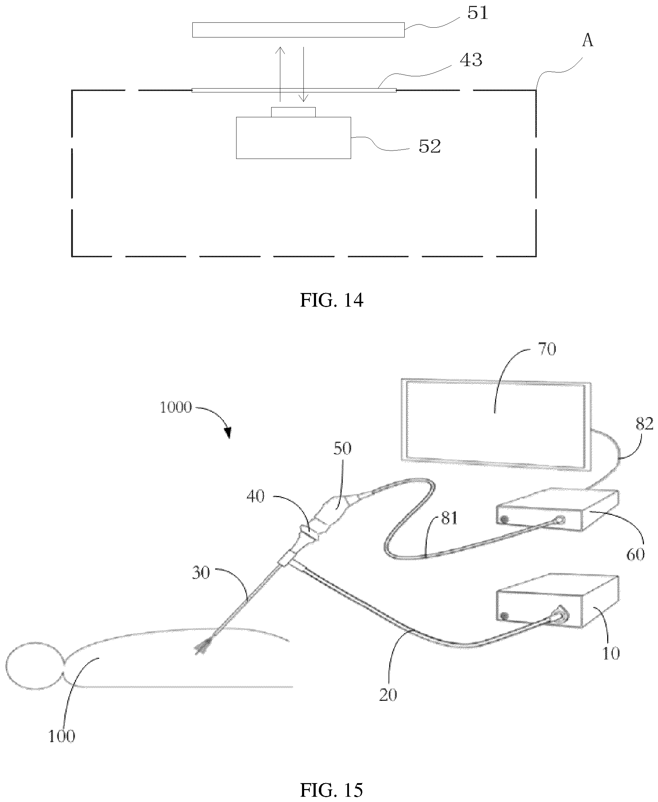

Please refer to FIG. 13. In an embodiment, the imaging head 50 is provided with a sealed cavity A. FIG. 14 shows a rough outline of the sealed cavity A by a dotted line. The dotted line is for reference only and is not an actual boundary of the sealed cavity A. The detection apparatus 5, the optical component 1 and the image sensor component 2 are all located inside the sealed cavity A, which can prevent external lights, water and other media from entering, and can protect a detection optical path and an imaging optical path inside the imaging head 50, as well as electrical connections and components between various components inside the imaging head 50. At the same time, the imaging head 50 can be sterilized and disinfected in an environment such as high-temperature immersion, thus satisfying requirements of sterilization and repeated use. Wherein, the imaging head 50 can be arranged with one or more sealed cavities A. The detection apparatus 5, the optical component 1 and the image sensor component 2 can be located inside one sealed cavity A. The detection apparatus 5, the optical component 1 and the image sensor component 2 can also be located in different sealed cavities A, respectively.

The handle 6, the front cover 8, the operation member 4, the first sealing member 42, the lens barrel 7, and other components of the imaging head 50 surround to form the sealed cavity A. The detection apparatus 5, the optical component 1 and the image sensor component 2 are located inside the sealed cavity A. Wherein, the sealed cavity A can be divided into three small cavities. The operation member 4, the first sealing member 42, and the lens barrel 7 surround a first small cavity, the detection apparatus 5 is located inside the first small cavity. The lens barrel 7 itself can surround a second small cavity, the optical component 1 is located inside the second small cavity. The handle 6, the front cover 8 and the lens barrel 7 surround a third small cavity, the image sensor component 2 is located inside the third small cavity. The three small cavities can be interconnected with each other, so as to satisfy connection requirements of circuits and components. The detection optical path and the imaging optical path are located inside a sealed cavity, so that external light cannot enter the detection optical path and the imaging optical path, and impurities such as water can be prevented from entering an environment surrounding the detection optical path and an environment surrounding the imaging optical path, thereby ensuring detection accuracy and imaging quality. Component, such as the image sensor component 2, is located inside the sealed cavity A, which can avoid short circuit caused by contact with water and failure due to high temperature. The driving apparatus 3 may also be inside the sealed cavity A, so as to provide water-proof and heat-insulating protection for the driving apparatus 3.

In an embodiment, when the optical triggering member 51 detects the rotation angle and the rotation direction of the operation member 4 by reflection, the optical triggering member 51 is located outside the sealed cavity A of the imaging head 50, and the optical detection member 52 is located inside the sealed cavity A of the imaging head 50. The optical triggering member 51 is a passive triggering structure, and the optical triggering member 51 does not involve a circuit connection structure. Therefore, the optical triggering member 51 is arranged outside the sealed cavity A, and will not be affected in a sterilization environment. The optical detection member 52 is provided with a light source and an electrical connection structure. The optical detection member 52 is arranged inside the sealed cavity A. The sealed cavity A can provide water-proof and heat-insulating protection for the optical detection member 52 .

Please refer to FIG. 14. A second sealing member 43 may be arranged between the optical triggering member 51 and the optical detection member 52. The second sealing member 43 may surround a part of the sealed cavity A. For example, the second sealing member 43 divides the mounting groove between the operation member 4 and the lens barrel 7 into two cavities, one of which is connected with outside, and the other cavity forms the sealed cavity A with a space inside the handle 6. The optical triggering member 51 is located inside the cavity which is connected with the outside, and the optical detection member 52 is located inside the sealed cavity A.

The second sealing member 43 may be a transparent or translucent structure. The detection light emitted by the optical detection member 52 can pass through the second sealing member 43 to illuminate the optical triggering member 51, and the detection light reflected by the optical triggering member 51 can also pass through the second sealing member 43 to illuminate the optical detection member 52 back. The light-transmitting setting of the second sealing member 43 ensures that, even if the second sealing member 43 separates the optical triggering member 51 and the optical detection member 52 in different cavities, the optical triggering member 51 and the optical detection member 52 can detect the rotation direction and the rotation angle of the operation member 4 by emitting and reflecting the detection light.

In an embodiment, the second sealing member 43 may also have a special structure, such as a non-transparent structure, and the second sealing member 43 can allow a designated light to pass therethrough. The detection light emitted by the optical detection member 52 includes the designated light, so that the detection light can also pass through the second sealing member 43 to achieve detection. If the designated light is an infrared light, the second sealing member 43 can allow the infrared light to pass through, the detection light emitted by the optical detection member 52 includes the infrared light, the optical triggering member 51 is configured to reflect the infrared light. The optical triggering member 51 and the optical detection member 52 detect the rotation angle and rotation direction of the operation member 4 by emitting and reflecting the infrared light.

As shown in FIG. 15, an imaging system for medical endoscope 1000 is provided in an embodiment. The imaging system for medical endoscope 1000 includes a light source 10, a light guide beam 20, a medical endoscope device, a communication cable 81, an imaging head host 60, a display 70, and a video connection cable 82. The medical endoscope device is the medical endoscope device in the above-mentioned embodiment.

The medical endoscope device includes an insertion portion 30 and an imaging head 50. The insertion portion 30 can be a rigid endoscope. The insertion portion 30 includes a front end and a rear end. The light source 10 is connected with the rear end of the insertion portion 30 through a light guide beam 20. The insertion portion 30 has an illumination channel and an imaging channel. An illumination light and/or excitation light emitted by the light source 10 passes through the light guide beam 20 and the illumination channel of the insertion portion 30 in turn and illuminate a region of a patient to be observed.

The imaging head host 60 is connected with the medical endoscope device via the communication cable 81, the image signal obtained by the medical endoscope device is transmitted to the imaging head host 60 via the communication cable 81 for processing. In some embodiments, the communication cable 81 can be an optical communication cable, such as an optical fibre. The medical endoscope device converts the image signal (electrical signal) into an optical signal, which is transmitted to the imaging head host 60 via the communication cable 81, and the imaging head host 60 then converts the optical signal back into an electrical signal. The imaging head host 60 is connected with the display 70 via the video connection line 82 for transmitting video signals to the display 70 for displaying. Those skilled in the art should understand that FIG. 15 is merely an example of the imaging system for medical endoscope 1000 and does not constitute a limitation of the imaging system for medical endoscope 1000. The imaging system for medical endoscope 1000 may include more or fewer components than shown in FIG. 15, or a combination of certain components, or different components.

The light source 10 is configured to provide an illumination source to a region of a patient to be observed 100. The illumination light source includes an illumination light source of visible light and a laser illumination light source (e.g., a near infrared light) corresponding to a fluorescent agent. The light source 10 includes, but is not limited to, a laser light source, an LED light source, or a laser diode.

In this embodiment, the light source 10 includes a visible light source and a laser light source corresponding to a fluorescent agent. The visible light source is an LED light source. In an embodiment, the visible light source can provide a plurality of monochromatic lights in different wavelength ranges, such as a blue light, a green light, a red light, etc. In other embodiments, the visible light source may also provide a combination of multiple monochromatic lights, or may be a broad-spectrum white light source. The wavelength of the monochromatic light is approximately in a range of 400 nm to 700 nm. The laser light source is configured to generate a laser light. The laser light is, for example, near infrared light (NIR). A peak wavelength of the laser takes at least one value within a range of 780 nm or 808 nm.

The light source 10 may provide continuous visible light and laser light corresponding to a fluorescent agent to the region to be observed simultaneously, or may provide two types of lights in a time-division manner.

Before imaging using the imaging system for medical endoscope 1000, a contrast agent, such as indocyanine green (ICG), is introduced into the region of the patient to be observed 100 by intravenous or subcutaneous injection to image tissue structures and functions (such as blood/lymph/bile in blood vessels) that are not easily visible, when using standard visible light imaging technology. The region of the patient to be observed 100 includes, but is not limited to, blood circulation system, lymphatic system, and tumour tissue. ICG, commonly known as indocyanine green, diagnostic green needle, and indocyanine green, is a contrast agent currently used in the clinical diagnosis of cardiovascular diseases and is widely used in choroidal and retinal vascular imaging. When the contrast agent in the region of the patient to be observed 100 absorbs a laser light, which is generated by the laser light source and corresponding to the fluorescent agent, fluorescence may be generated.

According to the imaging system for medical endoscope 1000 provided in the embodiment of this disclosure, since a driving apparatus 3 and a detection apparatus 5 are added in the medical endoscope device, an operation amount of the operation member 4 can be detected through the detection apparatus 5, the optical component 1 can be driven to focus through the driving apparatus 3 according to the detected operation amount. As the operation member 4 is a non-direct driving member, such that the rotation restriction of the operation member 4 is released. In this way, the operation member 4 can be rotated arbitrarily to indirectly drive the focusing, which greatly improves the use feel and increases the focusing range.

The above specific embodiments are used to illustrate this disclosure, which are only configured to help understand this disclosure and are not intended to limit this disclosure. For those skilled in the art of the technical field to which this disclosure belongs, several simple deductions, deformations or substitutions can be made according to the idea of this disclosure.

## Claims

1. A medical endoscope device, **characterized in that**, comprising:
an insertion portion (30), which comprises a front end and a rear end; wherein the front end of the insertion portion (30) is capable of being inserted into a region (100) of a patient to be observed, so as to acquire an image light which is reflected and/or excited by the region (100) of the patient to be observed; and
an imaging head (50), which comprises:
an optical component (1), wherein the optical component (1) and the rear end of the insertion portion (30) are optically butted;
an image sensor component (2), wherein the optical component (1) and the image sensor component (2) are movable relative to each other; the optical component (1) is configured to illuminate the image sensor component (2) with the image light which is acquired by the insertion portion (30); the image sensor component (2) is configured to obtain the image light and generate an image signal according to the image light, wherein the image signal is used to generate a visible image of the region (100) of the patient to be observed;
a driving apparatus (3), which is connected with the optical component (1) and/or the image sensor component (2), and is configured to drive at least one of the optical component (1) and the image sensor component (2) to move, so as to adjust a distance of an optical path between the optical component (1) and the image sensor component (2);
a rotatable operation member (4); and
a detection apparatus (5), which comprises an optical triggering member (51) and an optical detection member (52), wherein at least one of the optical triggering member (51) and the optical detection member (52), and the operation member (4) are linked together, so as to move together, and a rotation of the operation member (4) is capable of enabling a relative rotation between the optical triggering member (51) and the optical detection member (52);
the optical triggering member (51) is provided with a triggering area (511);
the optical detection member (52) is configured to emit a detection light and illuminate the triggering area (511) with the detection light; the triggering area (511) is configured to reflect or transmit the detection light; wherein during the rotation of the operation member (4), different positions of the triggering area (511) are illuminated by the detection light; the optical detection member (52) is further configured to receive the detection light which is reflected or transmitted by the triggering area (511), and generate different electrical signals based on the reflected or transmitted detection light; the driving apparatus (3) is further configured to drive at least one of the optical component (1) and the image sensor component (2) to move according to the different electrical signals, so as to adjust the distance of the optical path between the optical component (1) and the image sensor component (2) to achieve focusing.

2. The medical endoscope device according to claim 1, **characterized in that**, the different electrical signals are capable of at least being used to determine a rotation angle of the operation member.

3. The medical endoscope device according to claim 1, **characterized in that**, reflection or transmission of the detection light by the different positions of the triggering area (511) is capable of forming different reflected lights or different transmitted lights;
the optical detection member (52) is further configured to receive the different reflected lights or the different transmitted lights, and generate the different electrical signals based on the different reflected lights or the different transmitted lights.

4. The medical endoscope device according to claim 1, **characterized in that**, the triggering area (511) comprises a plurality of sub-triggering areas which are arranged along a circumferential direction, wherein adjacent sub-triggering areas have different light reflectivity or different light transmissivity.

5. The medical endoscope device according to claim 4, **characterized in that**, each sub-triggering area has a same width along the circumferential direction, and/or
each pair of adjacent sub-triggering areas has a same space.

6. The medical endoscope device according to claim 4, **characterized in that**, light reflectivity or light transmissivity of the plurality of sub-triggering areas increases or decreases along the circumferential direction.

7. The medical endoscope device according to claim 1, **characterized in that**, the triggering area (511) comprises a first sub-triggering area (5111) and a second sub-triggering area (5112) which are alternately arranged, wherein the first sub-triggering area (5111) and the second sub-triggering area (5112) have different light reflectivity or different light transmissivity.

8. The medical endoscope device according to claim 7, **characterized in that**, the optical detection member (52) is specifically configured to emit a first detection light and a second detection light, the electrical signals comprise a first electrical signal and a second electrical signal; the different positions of the triggering area (511) are simultaneously illuminated with the first detection light and the second detection light; reflection or transmission of the first detection light by the triggering area (511) forms a first reflected light or a first transmitted light, and reflection or transmission of the second detection light by the triggering area (511) forms a second reflected light or a second transmitted light; wherein the optical detection member (52) is specifically configured to receive the first reflected light or the first transmitted light and generate the first electrical signal, and to receive the second reflected light or the second transmitted light and generate the second electrical signal, wherein the first electrical signal and/or the second electrical signal are/is used to calculate a rotation angle of the operation member (4), wherein the first electrical signal and the second electrical signal are used to jointly determine a rotation direction of the operation member (4).

9. The medical endoscope device according to claim 8, **characterized in that**:
when the first sub-triggering area (5111) is illuminated with the first detection light, the second sub-triggering area (5112) is illuminated with the second detection light; and
when the second sub-triggering area (5112) is illuminated with the first detection light, the first sub-triggering area (5111) is illuminated with the second detection light.

10. The medical endoscope device according to claim 8, **characterized in that**, the first electrical signal comprises a first pulse signal, the second electrical signal comprises a second pulse signal, wherein a difference in pulse phase exists between the first pulse signal and the second pulse signal.

11. The medical endoscope device according to claim 10, **characterized in that**, the difference in pulse phase between the first pulse signal and the second pulse signal is 90°.

12. The medical endoscope device according to claim 8, **characterized in that**, the optical detection member (52) comprises a first optical detection member (521) and a second optical detection member (522);
wherein the first optical detection member (521) is configured to emit the first detection light, receive the first reflected light or the first transmitted light, and generate the first electrical signal; the second optical detection member (522) is configured to emit the second detection light, receive the second reflected light or the second transmitted light, and generate the second electrical signal.

13. The medical endoscope device according to claim 1, **characterized in that**, the triggering area has gradually changing light reflectivity or gradually changing light transmissivity along a circumferential direction.

14. The medical endoscope device according to claim 13, **characterized in that**, the gradually changing light reflectivity or the gradually changing light transmissivity is linearly changing light reflectivity or linearly changing light transmissivity.

15. The medical endoscope device according to claim 1, **characterized in that**, the optical triggering member (51) is provided with a side flat surface (51a), a central axis of the relative rotation between the optical triggering member (51) and the optical detection member (52) is perpendicular to the side flat surface (51a), the triggering area (511) is arranged on the side flat surface (51a); or
the optical triggering member (51) is provided with a cylindrical surface (51b), a central axis of the cylindrical surface (51b) overlaps with a central axis of the relative rotation between the optical triggering member (51) and the optical detection member (52), the triggering area (511) is arranged on the cylindrical surface (51b).

16. The medical endoscope device according to claim 1, **characterized in that**, the optical detection member (52) is provided with an emitting end (52a) and a receiving end (52b);
wherein the triggering area (511) is configured to reflect the detection light, the emitting end (52a) and the receiving end (52b) are located at a same side of the optical triggering member (51); and/or
the triggering area (511) is configured to transmit the detection light, the emitting end (52a) and the receiving end (52b) are located at respective sides of the optical triggering member (51).

17. The medical endoscope device according to claim 1, **characterized in that**, an environment surrounding an optical path of the optical component (1) and an environment surrounding an optical path of the detection apparatus (5) are isolated from each other.

18. The medical endoscope device according to claim 17, **characterized in that**, further comprising a lens barrel (7); wherein the optical component (1) is arranged inside the lens barrel (7), the environment surrounding the optical path of the optical component (1) is located at an inner side of the lens barrel (7), the environment surrounding the optical path of the detection apparatus (5) is located at an outer side of the lens barrel (7).

19. The medical endoscope device according to claim 18, **characterized in that**, the operation member (4) is located at the outer side of the lens barrel (7), and is capable of rotating relative to the lens barrel (7); wherein the operation member (4) and/or the lens barrel (7) are/is provided with an annular groove (41), and the detection apparatus (5) is located inside said annular groove (41).

20. The medical endoscope device according to claim 18, **characterized in that**, the operation member (4) is provided with an annular groove (41), the optical triggering member (51) is located at a side wall of said annular groove (41) along an axial direction, and the optical detection member (52) is arranged at the lens barrel (7).

21. The medical endoscope device according to claim 19 or claim 20, **characterized in that**, the annular groove (41) has an axial opening, wherein the axial opening is provided with a first sealing member (42).

22. The medical endoscope device according to claim 1, **characterized in that**, the imaging head (50) is provided with a sealed cavity (A), wherein the detection apparatus (5) is located inside said sealed cavity (A).

23. The medical endoscope device according to claim 22, **characterized in that**, the optical component (1) and the image sensor component (2) are located inside said sealed cavity (A).

24. The medical endoscope device according to claim 1, **characterized in that**, the imaging head (50) is provided with a sealed cavity (A), the optical triggering member (51) is located outside said sealed cavity (A), and the optical detection member (52) is located inside said sealed cavity (A).

25. The medical endoscope device according to claim 24, **characterized in that**, a second sealing member (43) is provided between the optical triggering member (51) and the optical detection member (52), the second sealing member (43) surrounds a part of said sealed cavity (A), wherein the detection light is capable of passing through the second sealing member (43).

26. The medical endoscope device according to claim 25, **characterized in that**, the second sealing member (43) is a transparent or translucent structure.

27. The medical endoscope device according to claim 25, **characterized in that**, the second sealing member (43) is a structure which is capable of allowing a designated light to pass therethrough, wherein the detection light comprises the designated light.

28. A medical endoscope device, **characterized in that**, comprising:
an insertion portion (30), which comprises a front end and a rear end; wherein the front end of the insertion portion (30) is capable of being inserted into a region (100) of a patient to be observed, so as to acquire an image light which is reflected and/or excited by the region (100) of the patient to be observed; and
an imaging head (50), which comprises:
an optical component (1), wherein the optical component (1) and the rear end of the insertion portion (30) are optically butted;
an image sensor component (2), wherein the optical component (1) and the image sensor component (2) are movable relative to each other; the optical component (1) is configured to illuminate the image sensor component (2) with the image light which is acquired by the insertion portion (30); the image sensor component (2) is configured to obtain the image light and generate an image signal according to the image light, wherein the image signal is used to generate a visible image of the region (100) of the patient to be observed;
a driving apparatus (3), which is connected with the optical component (1) and/or the image sensor component (2), and is configured to drive at least one of the optical component (1) and the image sensor component (2) to move, so as to adjust a distance of an optical path between the optical component (1) and the image sensor component (2);
a rotatable operation member (4); and
a fixation member (7, 21), wherein the operation member (4) is rotatable relative to the fixation member (7, 21), one of the operation member (4) and the fixation member (7, 21) is provided with a triggering area (511); and
a detection apparatus (5), which comprises an optical detection member (52), wherein the optical detection member (52) is arranged at the other of the operation member (4) and the fixation member (7, 21), and a rotation of the operation member (4) is capable of enabling a relative rotation between the triggering area (511) and the optical detection member (52);
the optical detection member (52) is configured to emit a detection light and illuminate the triggering area (511) with the detection light; the triggering area (511) is configured to reflect or transmit the detection light; wherein during the rotation of the operation member (4), different positions of the triggering area (511) are illuminated by the detection light; the optical detection member (52) is further configured to receive the detection light which is reflected or transmitted by the triggering area (511), and generate different electrical signals based on the reflected or transmitted detection light; the driving apparatus (3) is further configured to drive at least one of the optical component (1) and the image sensor component (2) to move according to the different electrical signals, so as to adjust the distance of the optical path between the optical component (1) and the image sensor component (2) to achieve focusing.

29. The medical endoscope device according to claim 28, **characterized in that**, the different electrical signals are capable of at least being used to determine a rotation angle of the operation member.

30. The medical endoscope device according to claim 28, **characterized in that**, reflection or transmission of the detection light by the different positions of the triggering area (511) is capable of forming different reflected lights or different transmitted lights;
the optical detection member (52) is further configured to receive the different reflected lights or the different transmitted lights, and generate the different electrical signals based on the different reflected lights or the different transmitted lights.

31. The medical endoscope device according to claim 28, **characterized in that**, the triggering area (511) comprises a plurality of sub-triggering areas which are arranged along a circumferential direction, wherein adjacent sub-triggering areas have different light reflectivity or different light transmissivity.

32. The medical endoscope device according to claim 31, **characterized in that**, light reflectivity or light transmissivity of the plurality of sub-triggering areas increases or decreases along the circumferential direction.

33. The medical endoscope device according to claim 28, **characterized in that**, the triggering area comprises a first sub-triggering area (5111) and a second sub-triggering area (5112) which are alternately arranged, wherein the first sub-triggering area (5111) and the second sub-triggering area (5112) have different light reflectivity or different light transmissivity.

34. The medical endoscope device according to claim 33, **characterized in that**, the optical detection member (52) is specifically configured to emit a first detection light and a second detection light, the electrical signals comprise a first electrical signal and a second electrical signal; the different positions of the triggering area (511) are simultaneously illuminated with the first detection light and the second detection light; reflection or transmission of the first detection light by the triggering area (511) forms a first reflected light or a first transmitted light, and reflection or transmission of the second detection light by the triggering area (511) forms a second reflected light or a second transmitted light; wherein the optical detection member (52) is specifically configured to receive the first reflected light or the first transmitted light and generate the first electrical signal, and to receive the second reflected light or the second transmitted light and generate the second electrical signal, wherein the first electrical signal and/or the second electrical signal are/is used to calculate a rotation angle of the operation member (4), wherein the first electrical signal and the second electrical signal are used to jointly determine a rotation direction of the operation member (4).

35. The medical endoscope device according to claim 34, **characterized in that**:
when the first sub-triggering area (5111) is illuminated with the first detection light, the second sub-triggering area (5112) is illuminated with the second detection light; and
when the second sub-triggering area (5112) is illuminated with the first detection light, the first sub-triggering area (5111) is illuminated with the second detection light.

36. The medical endoscope device according to claim 34, **characterized in that**, the first electrical signal comprises a first pulse signal, the second electrical signal comprises a second pulse signal, wherein a difference in pulse phase exists between the first pulse signal and the second pulse signal.

37. The medical endoscope device according to claim 28, **characterized in that**, the triggering area has gradually changing light reflectivity or gradually changing light transmissivity along a circumferential direction.

38. The medical endoscope device according to claim 37, **characterized in that**, the triggering area has linearly changing light reflectivity or linearly changing light transmissivity along the circumferential direction.

39. A medical endoscope device, **characterized in that**, comprising:
a rotatable operation member (4); and
a detection apparatus (5), which comprises an optical triggering member (51) and an optical detection member (52);
wherein at least one of the optical triggering member (51) and the optical detection member (52), and an operation member (4) are linked together, so as to move together, and a rotation of the operation member (4) is capable of enabling a relative rotation between the optical triggering member (51) and the optical detection member (52);
the optical triggering member (51) is provided with a triggering area (511);
the optical detection member (52) is configured to emit a detection light and illuminate the triggering area (511) with the detection light; the triggering area (511) is configured to reflect or transmit the detection light; wherein during the rotation of the operation member (4), different positions of the triggering area (511) are illuminated by the detection light; the optical detection member (52) is further configured to receive the detection light which is reflected or transmitted by the triggering area (511), and generate different electrical signals based on the reflected or transmitted detection light; wherein the different electrical signals are used for focusing of the medical endoscope device.

40. The medical endoscope device according to claim 39, **characterized in that**, the different electrical signals are capable of at least being used to determine a rotation angle of the operation member.

41. The medical endoscope device according to claim 39, **characterized in that**, reflection or transmission of the detection light by the different positions of the triggering area (511) is capable of forming different reflected lights or different transmitted lights;
the optical detection member (52) is further configured to receive the different reflected lights or the different transmitted lights, and generate the different electrical signals based on the different reflected lights or the different transmitted lights.

42. The medical endoscope device according to claim 39, **characterized in that**, the triggering area (511) comprises a plurality of sub-triggering areas which are arranged along a circumferential direction, wherein adjacent sub-triggering areas have different light reflectivity or different light transmissivity.

43. The medical endoscope device according to claim 42, **characterized in that**, light reflectivity or light transmissivity of the plurality of sub-triggering areas increases or decreases along the circumferential direction.

44. The medical endoscope device according to claim 39, **characterized in that**, the triggering area comprises a first sub-triggering area (5111) and a second sub-triggering area (5112) which are alternately arranged, wherein the first sub-triggering area (5111) and the second sub-triggering area (5112) have different light reflectivity or different light transmissivity.

45. The medical endoscope device according to claim 44, **characterized in that**, the optical detection member (52) is specifically configured to emit a first detection light and a second detection light, the electrical signals comprise a first electrical signal and a second electrical signal; the different positions of the triggering area (511) are simultaneously illuminated with the first detection light and the second detection light; reflection or transmission of the first detection light by the triggering area (511) forms a first reflected light or a first transmitted light, and reflection or transmission of the second detection light by the triggering area (511) forms a second reflected light or a second transmitted light; wherein the optical detection member (52) is specifically configured to receive the first reflected light or the first transmitted light and generate the first electrical signal, and to receive the second reflected light or the second transmitted light and generate the second electrical signal, wherein the first electrical signal and/or the second electrical signal are/is used to calculate a rotation angle of the operation member (4), wherein the first electrical signal and the second electrical signal are used to jointly determine a rotation direction of the operation member (4).

46. The medical endoscope device according to claim 45, **characterized in that**:
when the first sub-triggering area (5111) is illuminated with the first detection light, the second sub-triggering area (5112) is illuminated with the second detection light; and
when the second sub-triggering area (5112) is illuminated with the first detection light, the first sub-triggering area (5111) is illuminated with the second detection light.

47. The medical endoscope device according to claim 45, **characterized in that**, the first electrical signal comprises a first pulse signal, the second electrical signal comprises a second pulse signal, wherein a difference in pulse phase exists between the first pulse signal and the second pulse signal.

48. The medical endoscope device according to claim 39, **characterized in that**, the triggering area has gradually changing light reflectivity or gradually changing light transmissivity along a circumferential direction.

49. The medical endoscope device according to claim 48, **characterized in that**, the triggering area has linearly changing light reflectivity or linearly changing light transmissivity along the circumferential direction.

50. A medical endoscope device, **characterized in that**, comprising:
a rotatable operation member (4); and
a fixation member (7, 21), wherein the operation member (4) is rotatable relative to the fixation member (7, 21), one of the operation member (4) and the fixation member (7, 21) is provided with a triggering area (511); and
a detection apparatus (5), which comprises an optical detection member (52), wherein the optical detection member (52) is arranged at the other of the operation member (4) and the fixation member (7, 21) which is not provided with the triggering area (511), and a rotation of the operation member (4) is capable of enabling a relative rotation between the triggering area (511) and the optical detection member (52);
the optical detection member (52) is configured to emit a detection light and illuminate the triggering area (511) with the detection light; the triggering area (511) is configured to reflect or transmit the detection light; wherein the optical detection member (52) is further configured to receive the detection light which is reflected or transmitted by the triggering area (511), and generate different electrical signals based on the reflected or transmitted detection light; wherein the different electrical signals are used for focusing of the medical endoscope device.

51. The medical endoscope device according to claim 50, **characterized in that**, the different electrical signals are capable of at least being used to determine a rotation angle of the operation member.

52. The medical endoscope device according to claim 50, **characterized in that**, reflection or transmission of the detection light by the different positions of the triggering area (511) is capable of forming different reflected lights or different transmitted lights;
the optical detection member (52) is further configured to receive the different reflected lights or the different transmitted lights, and generate the different electrical signals based on the different reflected lights or the different transmitted lights.

53. The medical endoscope device according to claim 50, **characterized in that**, the triggering area (511) comprises a plurality of sub-triggering areas which are arranged along a circumferential direction, wherein adjacent sub-triggering areas have different light reflectivity or different light transmissivity.

54. The medical endoscope device according to claim 53, **characterized in that**, light reflectivity or light transmissivity of the plurality of sub-triggering areas increases or decreases along the circumferential direction.

55. The medical endoscope device according to claim 50, **characterized in that**, the triggering area comprises a first sub-triggering area (5111) and a second sub-triggering area (5112) which are alternately arranged, wherein the first sub-triggering area (5111) and the second sub-triggering area (5112) have different light reflectivity or different light transmissivity.

56. The medical endoscope device according to claim 55, **characterized in that**, the optical detection member (52) is specifically configured to emit a first detection light and a second detection light, the electrical signals comprise a first electrical signal and a second electrical signal; the different positions of the triggering area (511) are simultaneously illuminated with the first detection light and the second detection light; reflection or transmission of the first detection light by the triggering area (511) forms a first reflected light or a first transmitted light, and reflection or transmission of the second detection light by the triggering area (511) forms a second reflected light or a second transmitted light; wherein the optical detection member (52) is specifically configured to receive the first reflected light or the first transmitted light and generate the first electrical signal, and to receive the second reflected light or the second transmitted light and generate the second electrical signal, wherein the first electrical signal and/or the second electrical signal are/is used to calculate a rotation angle of the operation member (4), wherein the first electrical signal and the second electrical signal are used to jointly determine a rotation direction of the operation member (4).

57. The medical endoscope device according to claim 56, **characterized in that**:
when the first sub-triggering area (5111) is illuminated with the first detection light, the second sub-triggering area (5112) is illuminated with the second detection light; and
when the second sub-triggering area (5112) is illuminated with the first detection light, the first sub-triggering area (5111) is illuminated with the second detection light.

58. The medical endoscope device according to claim 56, **characterized in that**, the first electrical signal comprises a first pulse signal, the second electrical signal comprises a second pulse signal, wherein a difference in pulse phase exists between the first pulse signal and the second pulse signal.

59. The medical endoscope device according to claim 50, **characterized in that**, the triggering area has gradually changing light reflectivity or gradually changing light transmissivity along a circumferential direction.

60. The medical endoscope device according to claim 59, **characterized in that**, the triggering area has linearly changing light reflectivity or linearly changing light transmissivity along the circumferential direction.

61. An imaging system for medical endoscope, **characterized in that**, comprising a light source (10), a light guide beam (20), an imaging head host (60), a display (70), and the medical endoscope device according to any one of claims 1-54; wherein the light source (10) is connected with the insertion portion (30) through the light guide beam (20), an illumination light and/or an excitation light which are/is emitted by the light source (10) pass(es) through the light guide beam (20) and the insertion portion (30) in sequence to illuminate the region (100) of the patient to be observed; the imaging head host (60) is in signal connection with the imaging head (50) of the medical endoscope device and the display (70) respectively; the imaging head host (60) is configured to obtain the image signal, process the image signal, and then output the image signal to the display (70) for displaying.
